(19) Europäisches Patentamt
European Patent Office
Office européen des brevets

(11) Publication number: 0 199 432 B1

(12) # EUROPEAN PATENT SPECIFICATION

(45) Date of publication of patent specification: 29.05.91

(51) Int. Cl.5: G01N 33/68, G01N 31/22, G01N 30/20, G01N 27/26, C07D 209/62, C07D 491/056, C07D 487/04, C07K 5/06

(21) Application number: 86301267.0

(22) Date of filing: 21.02.86

The file contains technical information submitted after the application was filed and not included in this specification

(54) **Assaying method for primary amines using aromatic dialdehydes.**

(30) Priority: 04.03.85 US 707676

(43) Date of publication of application: 29.10.86 Bulletin 86/44

(45) Publication of the grant of the patent: 29.05.91 Bulletin 91/22

(84) Designated Contracting States: AT BE CH DE FR GB IT LI LU NL SE

(56) References cited:
EP-A- 0 138 092
DE-A- 2 102 985
FR-A- 2 534 029

CHEMOCAL ABSTRACTS, vol.101, no. 3, July 16, 1984, Columbus, Ohio, USA MIURA et al. "A fluorometric method for the specific determination of serum argine with 2,3-naphtalene-dicarbaldehyde" page 313, columns 1,2, abstracts no. 20064f

Biochemical Medicine 13, 303-306 (1975)

(73) Proprietor: OREAD LABORATORIES, INC.
1501 Wakarusa Drive
Lawrence Kansas 6047(US)

(72) Inventor: Higuchi, Takeru
2811 Schwartz Road
Lawrence Kansas 66044(US)
Inventor: Montigny, Pierre M.R.
2500 West Sixth Street
Lawrence Kansas 66044(US)
Inventor: Repta, Arnold J.
Route 6, Box 100N
Lawrence Kansas 66046(US)
Inventor: Stobaugh, John F.
3405 Riverview Road
Lawrence Kansas 66044(US)
Inventor: Sternson, Larry A.
464 Margo Lane
Berwyn Pennsylvania 19312(US)

(74) Representative: Pendlebury, Anthony et al
PAGE, WHITE & FARRER 54 Doughty Street
London WC1N 2LS(GB)

EP 0 199 432 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

Analytical Biochemistry 116, 223-226 (1981)

Brain Research 296, 156-159 (1984)

J. Chromatog. 380, 33-41(1986)

## Description

The present invention relates to the fluorometric and electrochemical detection of low levels of primary amines and more particularly, of biogenic and endogenous amines such as catechol amines, amino acids, and peptides, by chemically converting the non-fluorescing amine of interest to an entity which is amenable to both fluorescent and electrochemical detection. Such conversion is achieved by reacting fluorogenic reagents having an aromatic dialdehyde ring with the primary amine in the presence of a cyanide ion under mildly alkaline conditions.

Assaying techniques wherein a fluorogenic reagent is reacted with a substrate to form a fluorescing complex have been known for some time. One reagent used specifically for the derivatization of primary amines has been o-phthaldehyde (OPA) which is of the formula:

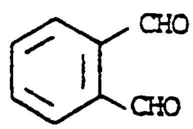

Under mildly alkaline conditions in the presence of a thiol, ($R_1SH$) and a primary amine ($R_2NH_2$), OPA forms 1-alkylthiol-2-alkylisoindole (AAI), which is a highly fluorescent compound of the formula:

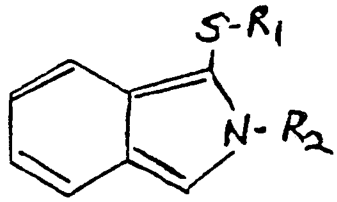

Although the fluorophores generated by the reaction of primary amines and OPA exhibit a relatively high fluorescent intensity with respect to many primary amines, it has been observed that the fluorescent intensity of the isoindole derivatives of primary amines containing an $\alpha$-amido group are substantially lower. Thus, the OPA/thiol derivatizing system is not useful for the detection of femtomole quantities of peptides and proteins. Such represents a significant drawback of OPA for assaying many biological systems.

Another problem encountered with fluorogenic assaying techniques employing OPA relates to the relative instability of the 1,2-disubstituted isoindoles of certain amines such as glycine, amino butyric acid (GABA), and B-alanine. These compounds have been observed to readily degrade into non-fluorescent products thereby placing severe time constraints on a practitioner when a concentration profile of the above amino acids is desired.

Naphthalene-2,3-dicarboxyaldehyde (NDA) of the formula:

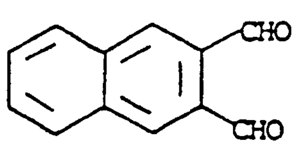

has also been used as a fluorogenic reagent (Analytical Biochemistry 139, 432-437(1984). However, its use has been limited to the detection of arginine and arginine methyl ester and, even in that limited case, the structure of the fluorescent reaction product has not been characterized.

In view of the foregoing limitations and shortcomings of prior art assaying techniques as well as other disadvantages not specifically mentioned above, it should be apparent that there still exists a need in the art for a method which enables assaying of trace concentrations of analytes containing one or more primary amino groups. It is, therefore, a primary objective to fulfill that need by providing a process wherein an aromatic dialdehyde is reacted with as little as $10^{-15}$ moles of primary amine in a solution to yield

compound which is detectable using fluorometric or electrochemical assaying techniques.

More particularly, it is an object of this invention to provide a process for assaying biogenic and endogenous amines such as catechol amines, amino acids, and peptides, such as glycyl-glycine and alanyl-alanine.

According to the present invention there is provided a method of assaying primary amines comprising the steps of:

(i) providing a substituted or unsubstituted naphthalene-2,3-dicarboxyaldehyde reactive with primary amines;

(ii) reacting said substituted or unsubstituted naphthalene-2,3-dicarboxyaldehyde with at least one primary amine analyte and cyanide ion to form compound amenable to fluorometric or electrochemical assaying techniques;

(iii) fluorometrically or eletrochemically assaying said compound.

The method of the present invention leads to the formation of a fluorescent compound which is amenable to detection by fluorometric and electrochemical techniques in amine concentrations as low as $10^{-12}$ moles/liter, and potentially even lower.

The compounds formed in the assaying process of the invention are highly stable. The compounds exhibit a high degree of fluorescence, and thus are amenable to fluorometric techniques. Furthermore, the compounds undergo anodic oxidation at moderate potential and thus are amenable to electrochemical detection techniques.

1. Preparation of Aromatic Dialdehydes

By the method of the present invention, a primary amine is reacted with an aromatic dialdehyde in the presence of cyanide ions. The aromatic dialdehydes may be selected from either substituted or unsubstituted naphthalene-2,3-dicarboxyaldehydes, and particularly those of the formula:

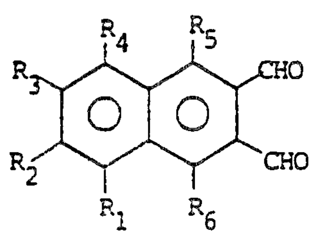

wherein:

(A) $R_1$ is H, $N(CH_3)_2$, $SO_3H$, $NO_2$, or $SO_3^{\ominus}Na^{\oplus}$, and $R_2$, $R_3$, $R_4$, $R_5$, and $R_6$ are H; or

(B) $R_1$, $R_4$, $R_5$, and $R_6$ are H, and $R_2$ and $R_3$ together are

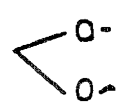

or

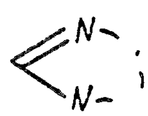

or

(C) $R_1$ and $R_4$ are $N(CH_3)_2$ and $R_2$, $R_3$, $R_5$, and $R_6$ are H;

or

(D) $R_1$, $R_2$, $R_3$, and $R_4$ are H and $R_5$ and $R_6$ are $OCH_3$,

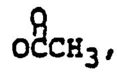

$OSi(CH_3)_2\ C_4H_9$, or $N(CH_3)_2$; or

(E) $R_1$, $R_4$, $R_5$, and $R_6$ are H and $R_2$ and $R_3$ are $OCH_3$, $SO_3H$, or $CO_2H$, or the salts thereof; or

(F) $R_1$, $R_3$, $R_4$, $R_5$, and $R_6$ are H and $R_2$ is $(CH_3)_2N$.

Unsubstituted naphthalene-2,3,-dicarboxyaldehyde of the formula:

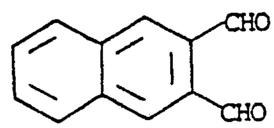

is a known compound and was prepared by the following alternative sequences:

(I)

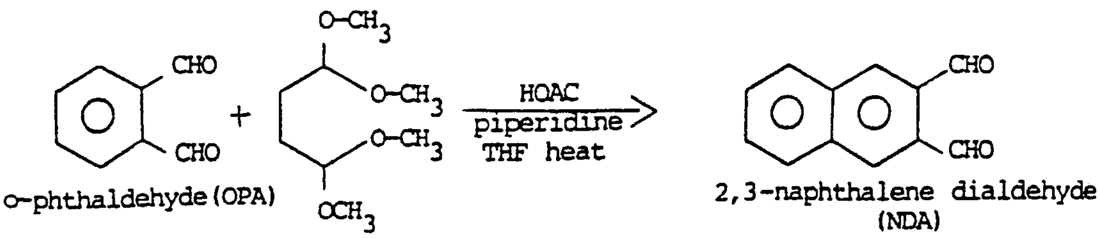


o-phthaldehyde (OPA)

2,3-naphthalene dialdehyde (NDA)

(II)

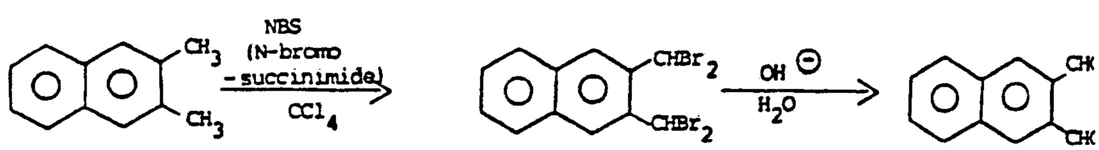


The latter process is reported by W. Ried and H. Bodem in Chem. Ber., Vol. 89, p. 708 (1956).

The substituted 2,3-naphthalene dialdehydes are prepared by forming a suitably substituted o-phthaldehyde for use in reaction (I) above. Thus, $R_1$, $R_2$, $R_3$, and $R_4$ substituents of the NDA can be selected by reacting similarly substituted o-xylene or phthalic anhydride as precursors to o-phthaldehyde according to the following sequences:

(III)

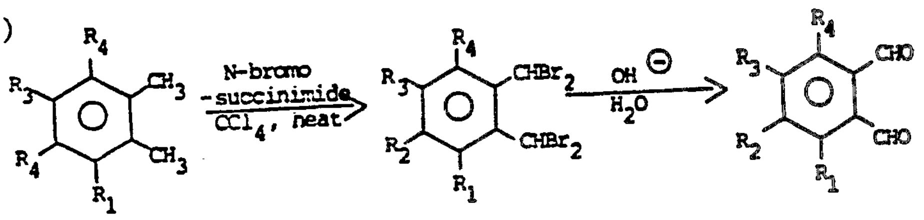
N-bromo
-succinimide
CCl4, heat
OH⊖
H2O

(IV)

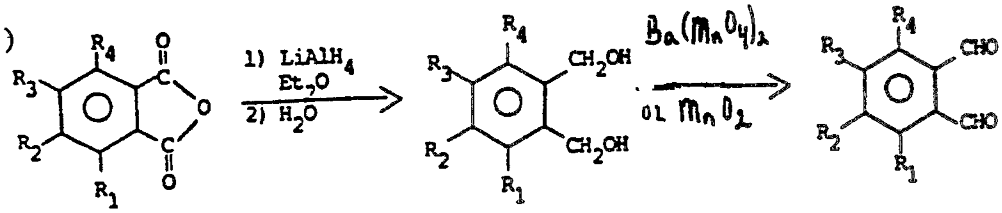
1) LiAlH4
Et2O
2) H2O
Ba(MnO4)2
or MnO2

(v)

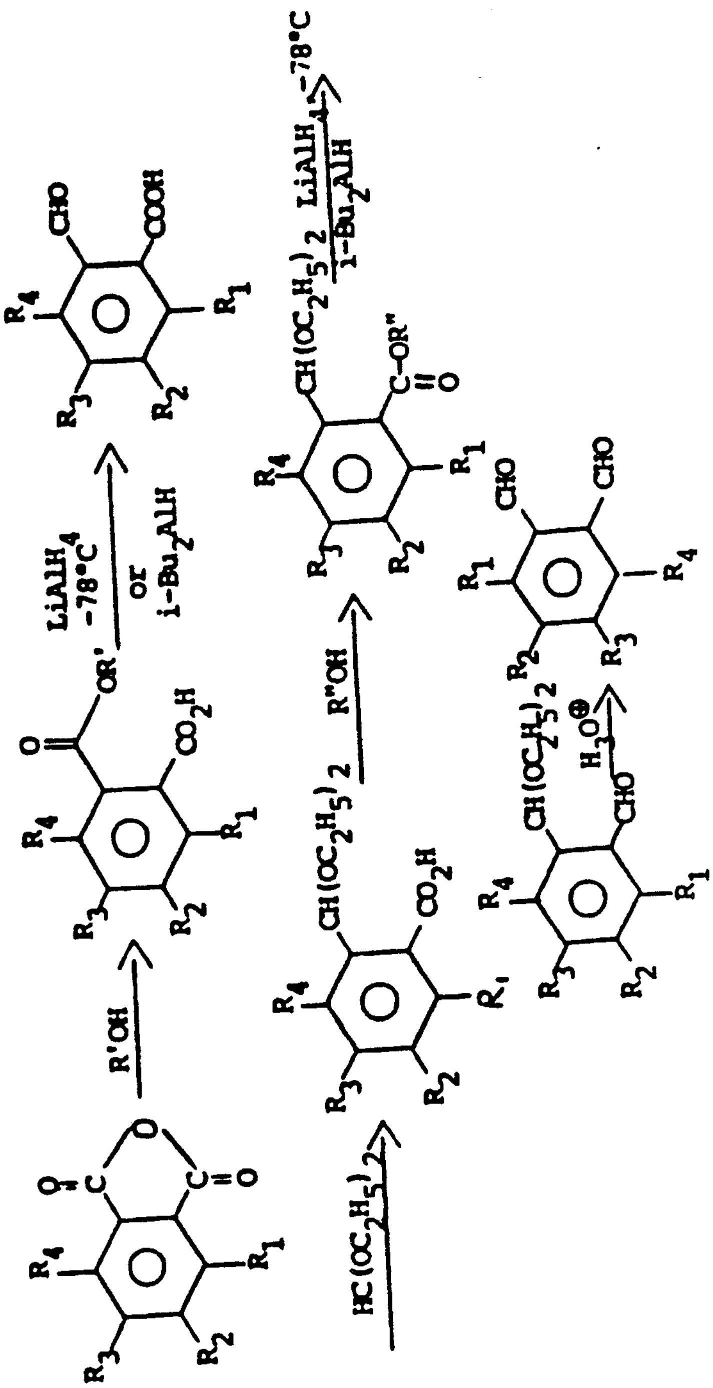


2. Formation of the compounds Amenable to Fluorometric or Electrochemical assaying Techniques

The aromatic dialdehydes were reacted with the primary amine analytes in the presence of cyanide ion ($CN^-$), or a precursor thereof, in a mild alkaline aqueous medium to give a highly stable compound which is intensely fluorescent and is electroactive at moderate potential. Use of the inventive compounds enables

detection of the described class of analytes in the femtomole range and beyond.

The reaction of the aromatic dicarboxyaldehyde was typically carried out at room temperature or 30° C at a pH from 9 to 10. Generally, a 100 to 200 micromolar solution of NDA was combined with a 100 to 200 micromolar solution of NaCN in the presence of a 10 to 100 millimolar borate buffer solution. For this NDA/CN system, the excitation and emission wavelengths were 420 nm and 490nm, respectively. These conditions are merely illustrative, and one skilled in the art will appreciate that conditions may be varied without affecting the compound formation.

When the above compounds were fluorometrically analyzed, it was observed that assaying could be carried out with as little as 50 femtomoles of analyte.

The compounds of the present invention are also amenable to electrochemical analytical techniques.

The highly fluorescent compounds of the present invention are formed by reacting the aromatic dialdehyde with the primary amine and cyanide by the following sequence:

(VI)

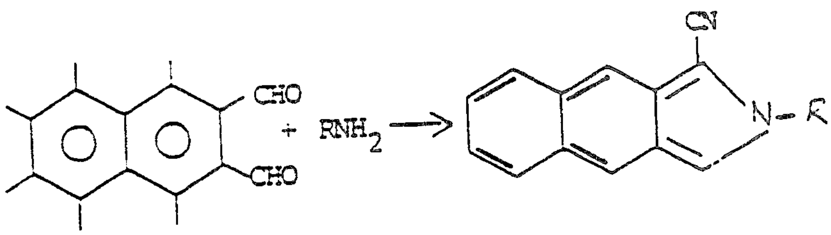

When the NDA/CN compounds are formed with a mixture of primary amines before the separating step, such as HPIC, it is possible that two peaks will be observed for each primary amine analyte due to the formation of isomers as shown by the following:

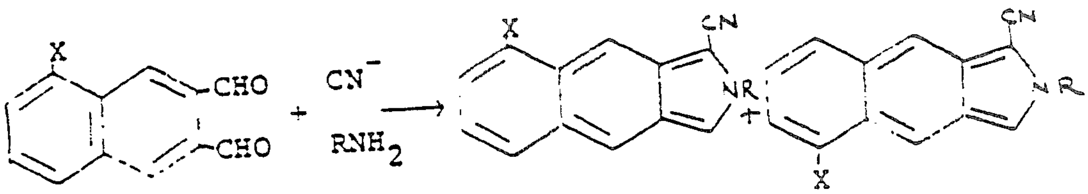

Since it is undesirable to obtain two peaks for each analyte in the chromotographic system, it is preferred to maintain the symmetry of the fluorogenic reagents through di- or tetrasubstitution wherein $R_1$ and $R_4$, $R_2$ and $R_3$, and $R_5$ and $R_6$ are identical.

The method of the present invention may be performed in the presence of a plurality of primary amines and may further comprise the step of separating the plurality of primary amines into individual amines preferably by high performance liquid chromatography. The step of separating may be carried out before or after reacting the substituted or unsubstituted naphthalene-2,3-dicarboxyaldehyde. Where appropriate, the method may use a 2,3-naphthalene-dicarboxyaldehyde which is di-substituted or tetrasubstituted.

The invention will be further clarified by a consideration of the following examples.

Example 1: 16 amino acids derivatized with 2-3-naphthalene dialdehyde

Into a low actinic 10m (volumetric flask) were added 9.6 ml of a 0.1 molar solution of sodium borate followed by 100 microliters of a $10^{-4}$ M solution of the following 16 amino acids:

glycine
alanine
tyrosine
valine
phenylalanine
aspartic acid
serine

glutamic acid
histidine
threonine
isoleucine
methionine
tryptophan
arginine
asparagine
glutamine

Next, 100 microliters of 0.05 M NaCN and 200 microliters of 0.01 M NDA in methanol were added. The solution was thoroughly mixed and placed in a 25° C. water bath.

A Schoeffel detector was used to measure fluorescence. The excitation wavelength was 420 nm and the emission wavelength 470 nm. An ODS 5μ column van also used. The gradient was 15-55% $CH_3CN$ in phosphate buffer. Solvent A (Mobile Phase) was 15% $CH_3CN$ and 85% 0.05 M $PO_4$ at a pH of 6.8. Solvent B was 55% $CH_3CN$ and 45% 0.05 M $PO_4$ at 9 pH of 6.8.

A chromatogram of the mixture was taken after 265 and 360 minutes and is shown at Fig. 1. The profiles after such time periods demonstrate the stabilities of the compounds of the present invention.

Example 2: Aspartame Derivatized With 2,3-Naphthalene Dicarboxyaldehyde

A solution containing about 1 mg/ml of NDA-CN-aspartame in borate buffer at a pH of 9.5 was analyzed by HPLC-UV (254nm) using a Waters 10μ column, an injection volume of 20 microliters, and a mobile phase comprised of 30% $CH_3CN$ in $H_2O$. Two peaks were observed (See Fig. 2), one of which increased with time and the other of which decreased with time. Without being limited by theory, it is believed that hydrolysis of the methyl ester of the dipeptide occurs since the second peak (the most hydrophobic) is the one decreasing with time.

Example 3: Dependence of the Observed Reaction Rate Constant ($k_{obs}$) on the Effective Concentration of Cyanide on NDA

A $2.0 \times 10^{-4}$ M solution of NDA was combined with a $2.0 \times 10^{-6}$ M solution of alanine in the presence of varying concentrations of cyanide ion. The pH of the mixture was 9.5 in a borate buffer at 25° C. and an ionic strength of 0.1. A graph of this relationship is shown in Fig. 3. The same determination of $k_{obs}$ was made with respect to the NDA concentration. Once again, the pH was 9.5, the reaction temperature 25° C., and the ionic strength 0.1. However, the concentration of cyanide and alanine were $1.0 \times 10^{-3}$ M and $2.0 \times 10^{-6}$ M respectively. The relationship is shown at Fig. 4.

Example 4: Determination of the Relative Fluorescent Intensities for the Analysis of Alanine With NDA in the Presence of Cyanide

In a quartz cuvette were added 100 microliters of 60 micromolar alanine solution followed by 100 microliters of 0.01 M NDA and 0.1 M NaCN in water. The relative fluorescent intensities of the exitation and emission spectra are shown in Figs. 5 and 6 respectively.

Example 5: Determination of the optimum pH for the NDA/CN Reaction with Alanine

Table I below lists the first-order rate constants, $k_{obs}$, for the formation of the NDA/CN-alanine product as a function of pH. A plot of $k_{obs}$ versus pH shows a maximum reaction rate at pH 9.5 (Fig. 7) which is analogous to the o-phthaldehyde 2 mercaptoethanol system. The reaction was carried out at 30° C. The concentrations of NDA, $CN^-$, and alanine were $2.0 \times 10^{-4}$ M, $2.0 \times 10^{-4}$ M and $1.0 \times 10^{-6}$ M, respectively.

TABLE I

First-order rate constants for the reaction of NDA with alanine and cyanide at 30° C, [NDA] = 0.20 mM, [CN] = 0.20 mM and [Ala] = 1.0 uM, pH maintained by pH-stat, ionic strength = 0.1.

| pH | $10^4 k_{obs}$, $s^{-1}$ |
|---|---|
| 8.0 | 4.9, 3.5 |
| 8.5 | 11.2, 10.6 |
| 9.0 | 22.3, 21.4 |
| 9.5 | 24.4, 22.4 |
| 10.0 | 8.9, 10.4 |
| 10.5 | 5.5, 3.6 |
| 11.0 | 2.0, 1.1 |

Example 6: Influence of Reaction Time Before Injection on the Fluorescence Intensity of 4 Amino Acids

A $10^{-4}$ M solution of glycine, β-alanine, GABA, and N-acetyllysine were reacted with a 0.01 M solution of NDA and 0.05 M CN to yield in 0.1 M borate buffer at pH 9.3, $10^{-6}$ M of the fluorophores. 50 microliters were then injected into HPLC device, with an R value of 0.5 μA. Separation using a linear gradient over 30 min from 15% $CH_3CN$ to 55% $CH_3CN$ in 0.05 M $PO_4$ at a pH of 6.8. The elution profiles after 15 minutes and 3 hours are shown in Fig. 8. As can be seen from Fig. 8, little or no degradation of the reaction products of the four amino acids with NDA/CH was observed after 3 hours at a pH of 9.3.

Example 7: Determination of the Detection Limit for the Analysis of 16 Amino Acids With NDA/CN

50 microliters of $10^{-4}$ M of a mixture of 16 amino acids were derivatized by reacting them with 50 microliters of 0.05 M NaCN and 100 μ1 of 0.01 M NDA for 2 minutes. This solution mixture was then diluted to give a $10^{-8}$ M solution of amino acids. A 50 microliter aliquot of the solution was then injected into the HPLC, i.e., 500 femtomoles. The column was ODS-hypersil, 5 μm particles, 4.6 x 150 mm. The guard column was 4.6 x 150 mm. The mobile phases were (A) 15% $CH_3CN$/85% $PO_4$, 0.05 M, pH 6.8 and (B) 55% $CH_3CN$/45% $PO_4$, 0.05 M, pH 6.8. The gradient was linear 100% A to 100% B in 60 min. The flow rate was 1.0 ml/min. A Schoeffel Detector FS970 was used. The exitation wavelength was 246 nm and the emission wavelength 470 nm. The time constant was 6s and the range was 0.2 μA. The chromatograph is shown at Fig. 9.

Comparative Example 1:

500 femtomoles of the reaction product o-phthaldehyde/mercaptoethanol and the same 16 amino acids analyzed in Example 7 were likewise subjected to an identical HPLC separation. The concentration of OPA was 1.8 x $10^{-6}$ whereas the concentration of mercapto-ethanol was 10 x $10^{-6}$ M. The reaction was carried out in a 0.1 M sodium borate solution and the products fluorometrically analyzed at an excitation wavelength of 230 nm and an emission wavelength of greater than 418 nm. The chromatogram is shown at Fig. 10. It will be appreciated that in contrast to the inventive compounds, the resolution of the OPA/2ME-amino acid reaction products is not nearly as great.

Comparative Example 2:

The degradation profiles of (A) 1-cyano-2-glycylbenzo-isoindole and (B) 1-mercaptoethanol 2-glycyl isoindole were compared. The compounds were analyzed in a mixed solvent, protected from light, consisting of 20% methanol and 80% borate buffer ( = 0.1 M, pH = 9.5). The mixture was maintained at 25° C. and monitored by HPLC. As shown in Fig. 11, the compounds of the present invention are far more stable than those obtained with OPA.

COMPARATIVE EXPERIMENT 3:

The detection limits of OPA-ME versus NDA-CN were again compared. An LKB 2151 variable wavelength monitor (Absolute wavelength of 335 nm for OPA and 420 nm for NDA); a Farrand optical spectrofluorometer (OPA excitation and emission wavelengths of 335 and 430 nm and NDA excitation and emission wavelengths of 420 and 480 nm) and a Rheodyne injector (20 microliter loop) were used. An LKB 2152 HPLC controller, an LKB 2150 HPLC pump and an LKB 2210 HPLC recorder were also used. The mobile phase for the OPA/ME products was 18% $CH_3CN$ : 82% 0.05M $PO_4$ at a pH of 6.8. The mobile phase for the NDA/CN products was 25% $CH_3CN$ : 75% 0.05M $PO_4$ and a pH of 6.8. The results are shown in Figs. 12 and 13 where a 20 picomole injection of glycine, alanine, and alanyl-alanine admixed with OPA/ME at pH 9.5, excitation wavelength of 335 nm, and emission wavelength of 430 nm and an R value = 0.01 are compared with 4 picomole injection of the same amino acids admixed with NDA/CN at pH 9.5, with an excitation wavelength of 420 nm and an emission wavelength of 480 nm and an R value of 0.01.

Thus, in terms of detectability and stability, the NDA/CN system of the present invention is far superior to the OPA/thiol system of the prior art.

## Claims

1. A method of assaying primary amines comprising the steps of:
   (i) providing a substituted or unsubstituted naphthalene-2,3-dicarboxyaldehyde reactive with primary amines;
   (ii) reacting said substituted or unsubstituted naphthalene-2,3-dicarboxyaldehyde with at least one primary amine analyte and cyanide ion to form a compound amenable to fluorometric or electrochemical assaying techniques;
   (iii) fluorometrically or electrochemically assaying said compound.

2. A method according to claim 1, wherein said compound is a fluorophore.

3. A method according to claim 1 or 2, wherein said substituted or unsubstituted naphthalene-2,3-dicarboxyaldehyde is of the formula:

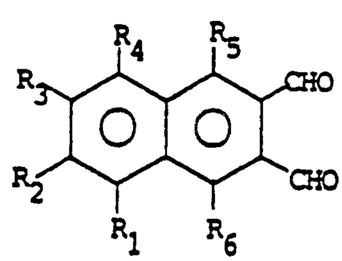

wherein
   (A) $R_1$ is H, $N(CH_3)_2$, $SO_3H$, $NO_2$, or $SO_3^{\ominus}$ $Na^{\oplus}$; and $R_2$, $R_3$, $R_4$, $R_5$, and $R_6$ are H; or
   (B) $R_1$, $R_4$, $R_5$, and $R_6$ are H, and $R_2$ and $R_3$ together are

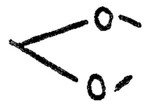

or

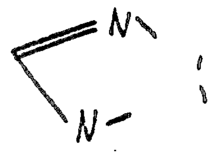

or

(C) $R_1$ and $R_4$ are $N(CH_3)_2$ and $R_2$, $R_3$, $R_5$, and $R_6$ are H;

or

(D) $R_1$, $R_2$, $R_3$, and $R_4$ are H and $R_5$ and $R_6$ are $OCH_3$,

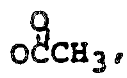

$OSi(CH_3)_2\ C_4H_9$, or $N(CH_3)_2$; or

(E) $R_1$, $R_4$, $R_5$, and $R_6$ are H and $R_2$ and $R_3$ are $OCH_3$, $SO_3H$ or $CO_2H$ or the salts thereof; or

(F) $R_1$, $R_3$, $R_4$, $R_5$, and $R_6$ are H and $R_2$ is $(CH_3)_2N$.

4. A method according to claim 3, wherein said substituted or unsubstituted naphthalene-2,3 dicarboxyaldehyde is of the formula:

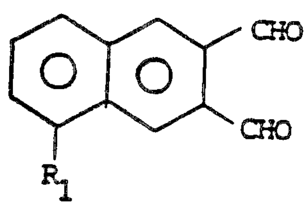

wherein $R_1$ is H, $N(CH_3)_2$, $SO_3H$, $NO_2$, or $SO_3$ Na .

5. A method according to claim 1, wherein said substituted or unsubstituted naphthalene-2,3 dicarboxyaldehyde is of the formula:

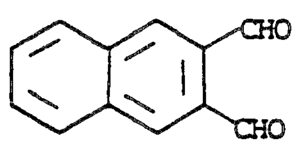

6. A method according to any one of the preceding claims, wherein a 100 to 200 micromolar solution of said substituted or unsubstituted naphthalene-2,3 dicarboxyaldehyde is reacted with a 100 to 200

micromolar solution of cyanide ion.

7. A method according to any one of the preceding claims, wherein said reacting step is carried out at a pH of from 9 to 10.

8. A method according to any one of the preceding claims, wherein a plurality of primary amines are present.

9. A method according to Claim 8, further comprising the step of separating said plurality of primary amines into individual amines.

10. A method according to Claim 9, wherein said separating step is carried out by high performance liquid chromatography.

11. A method according to Claim 8 or 9, wherein said separating step is carried out before said reacting step.

12. A method according to Claim 8 or 9, wherein said separating step is carried out after said reacting step.

13. A method according to any one of Claims 1 to 3 and 6 to 12, wherein said 2,3-naphthalene-dicarboxyaldehyde is di-substituted or tetrasubstituted.

14. A compound of the formula:

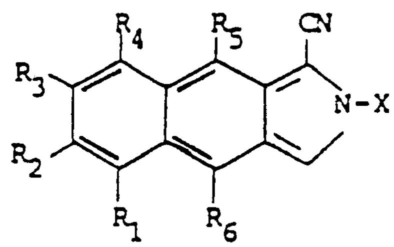

(A)

wherein X is a radical derived from a primary amine of the formula $X\text{-}NH_2$ and wherein:
(A) $R_1$ is H, $N(CH_3)_2$, $SO_3H$, $NO_2$, or $SO^{\ominus}_3\ Na^{\oplus}$, and $R_2$, $R_3$, $R_4$, $R_5$ and $R_6$ are H; or
(B) $R_1$, $R_4$, $R_5$, and $R_6$ are H, and $R_2$ and $R_3$ together are

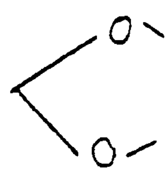

or

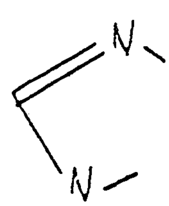

;or

(C) $R_1$ and $R_4$ are $N(CH_3)_2$ and $R_2$, $R_3$, $R_5$, and $R_6$ are H; or

(D) $R_1$, $R_2$, $R_3$, and $R_4$ are H and $R_5$ and $R_6$ are $OCH_3$,

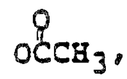

$OSi(CH_3)_2\ C_4H_9$, or $N(CH_3)_2$; or

(E) $R_1$, $R_4$, $R_5$, and $R_6$ are H and $R_2$ and R3 are $SO_3H$ or $CO_2H$ or the salts thereof; or

(F) $R_1$, $R_3$ $R_4$, $R_5$ and $R_6$ are H and $R_2$ is $(CH_3)2^{N.}$

**15.** A compound according to Claim 14, wherein $R_1$ is H, $N(CH_3)_2$, $SO_3H$,$NO_2$, or $SO_3^-Na^+$, and $R_2$, $R_3$, $R_4$, $R_5$ and $R_6$ are H.

**16.** A compound according to Claim 14, wherein each of $R_1$, $R_2$, $R_3$, $R_4$, $R_5$ and $R_6$ is a hydrogen atom.

**17.** A compound according to Claim 14, 15 or 16, wherein said primary amine of the formula $XNH_2$ is a peptide, an amino acid, or a catechol amine.

**18.** A compound according to Claim 14, 15 or 16, wherein said primary amine of the formula $XNH_2$ is glycine, alanine, tyrosine, valine, phenylalanine, aspartic acid, serine, glutamic acid, histidine, threonine, isoleucine, methionine, tryptophan, arginine, asparagine, B-alanine, GABA, N-Acetyl-lysine, glycyl-glycine, alanyl-alanine or aspartame.

Claims for the following Contracting States: AUSTRIA

**1.** A method of assaying primary amines comprising the steps of:

(i) providing a substituted or unsubstituted napthalene-2,3-dicarboxyaldehyde reactive with primary amines;

(ii) reacting said substituted or unsubstituted napthalene-2,3-dicarboxyaldehyde with at least one primary amine analyte and cyanide ion to form a compound amenable to fluorometric or electrochemical assaying techniques;

(iii) fluorometrically or electrochemically assaying said compound.

**2.** A method according to claim 1, wherein said compound is a fluorophore.

**3.** A method according to claim 1 or 2, wherein said substituted or unsubstituted napthalene-2,3-dicarboxyaldehyde is of the formula:

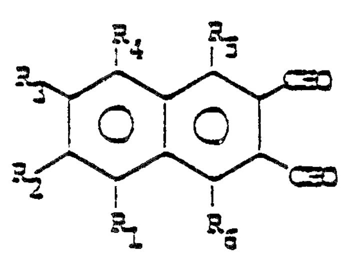

wherein

(A) R· is H, $N(CH_3)_2$, $SO_3H$, $NO_2$, or $SO_3^{\ominus}Na^{\oplus}$; and $R_2$, $R_3$, $R_4$, $R_5$, and $R_6$ are H; or

(B) $R_1$, $R_4$, $R_5$, and $R_6$ are H, and $R_2$ and $R_3$ together are

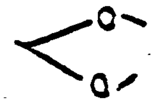

or

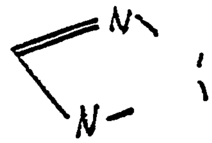

or

(C) $R_1$ and $R_4$ are $N(CH_3)_2$ and $R_2$, $R_3$, $R_5$, and $R_6$ are H; or

(D) $R_1$ $R_2$, $R_3$, and $R_4$ are H and $R_5$ and $R_6$ are $OCH_3$,

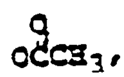

$OSi(CH_3)_2$ $C_4H_9$, or $N(CH_3)_2$; or

(E) $R_1$, $R_4$, $R_5$, and $R_6$ are H and $R_2$ and $R_3$ are $OCH_3$, $SO_3H$ or $CO_2H$ or the salts thereof; or

(F) $R_1$, $R_3$, $R_4$, $R_5$, and $R_6$ are H and $R_2$ is $(CH_3)\ _2N$.

**4.** A method according to claim 3, wherein said substituted or unsubstituted naphthalene-2,3 dicarboxyaldehyde is of the formula:

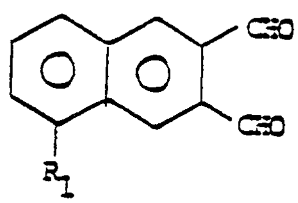

wherein $R_1$ is H, $N(CH_3)_2$, $SO_3H$, $NO_2$, or $SO_3$ Na .

**5.** A method according to claim 1, wherein said substituted or unsubstituted naphthalene-2,3 dicarboxyaldehyde is of the formula:

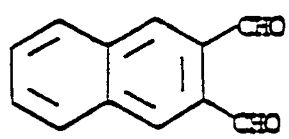

**6.** A method according to any one of the preceding claims, wherein a 100 to 200 micromolar solution of said substituted or unsubstituted naphthalene-2,3 dicarboxyaldehyde is reacted with a 100 to 200 micromolar solution of cyanide ion.

7. A method according to any one of the preceding claims, wherein said reacting step is carried out at a pH of from 9 to 10.

8. A method according to any one of the preceding claims, wherein a plurality of primary amines are present.

9. A method according to Claim 8, further comprising the step of separating said plurality of primary amines into individual amines.

10. A method according to Claim 9, wherein said separating step is carried out by high performance liquid chromatography.

11. A method according to Claim 8 or 9, wherein said separating step is carried out before said reacting step.

12. A method according to Claim 8 or 9, wherein said separating step is carried out after said reacting step.

13. A method according to any one of Claims 1 to 3 and 6 to 12, wherein said 2,3-naphthalene-dicarboxyaldehyde is di-substituted or tetrasubstituted.

14. A method of making a compound of general formula

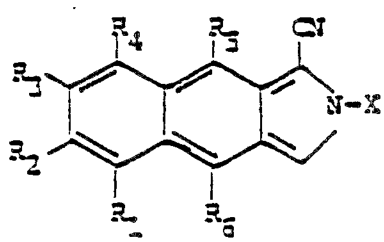

wherein X is a radical derived from a primary amine of the formula $X\text{-}NH_2$ and wherein:

(A) $R_1$ is H, $N(CH_3)_2$, $SO_3H$, $NO_2$, or $SO_3^-Na^+$, and $R_2$, $R_3$, $R_4$, $R_5$ and $R_6$ are H; or

(B) $R_1$, $R_4$, $R_5$, and $R_6$ are H, and $R_2$ and $R_3$ together are

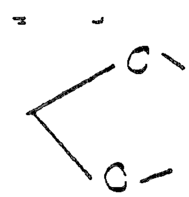

or

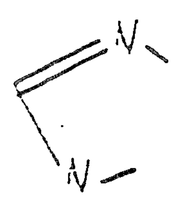

; or

(C) $R_1$ and $R_4$ are $N(CH_3)_2$ and $R_2$, $R_3$, $R_5$, and $R_6$ are H;

or

(D) $R_1$, $R_2$, $R_3$, and $R_4$ are H and $R_5$ and $R_6$ are $OCH_3$,

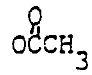

$OSi(CH_3)_2$ $C_4H_9$, or $N(CH_3)_2$; or
(E) $R_1$, $R_4$, $R_5$, and $R_6$ are H and $R_2$ and $R_3$ are $OCH_3$ $SO_3H$ or $CO_2H$ or the salts thereof; or
(F) $R_1$, $R_3$, $R_4$, $R_5$ and $R_6$ are H and $R_2$ is $(CH_3)_2N$, which method comprises reacting an aromatic dialdehyde of general formula

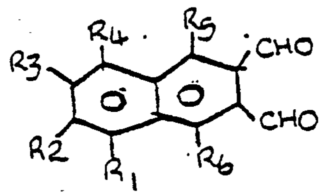

with the primary amine in the presence of cyanide.

**15.** A method according to Claim 14, wherein $R_1$ is H, $N(CH_3)_2$, $SO_3H$, $NO_2$, or $SO_3^- Na^+$, and $R_2$, $R_3$, $R_4$, $R_5$ and $R_6$ are H.

**16.** A method according to Claim 14, wherein each of $R_1$, $R_2$, $R_3$, $R_4$, $R_5$ and $R_6$ is a hydrogen atom.

**17.** A method according to Claim 14, 15 or 16, wherein said primary amine of the formula $XNH_2$ is a peptide, an amino acid, or a catechol amine.

**18.** A method according to Claim 14, 15 or 16, wherein said primary amine of the formula $XNH_2$ is glycine, alanine, tyrosine, valine, phenylalanine, aspartic acid, serine, glutamic acid, histidine, threonine, isoleucine, methionine, tryptophan, arginine, asparagine, B-alanine, GABA, N-Acetyl-lysine, glycyl-glycine, analyl-alanine, or aspartame.

## Revendications

**1.** Procédé de dosage d'amines primaires comprenant les étapes suivantes :
(i) prévoir un naphtalène-2,3-dicarboxyaldéhyde substitué ou non substitué réactif avec des amines primaires;
(ii) faire réagir le naphtalène -2,3-dicarboxyaldéhyde substitué ou non substitué avec au moins un analyte d'amine primaire et un ion cyanure pour former un composé pouvant être soumis à des techniques de dosage fluorimétrique ou électrochimique;
(iii) doser fluorimétriquement ou électrochimiquement ce composé.

**2.** Procédé suivant la revendication 1, caractérisé en ce que ce composé est un fluorophore.

**3.** Procédé suivant l'une ou l'autre des revendications 1 et 2, caractérisé en ce que le naphtalène-2,3-dicarboxyaldéhyde substitué ou non substitué est de la formule :

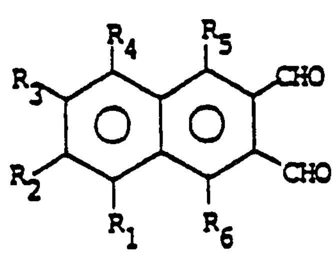

dans laquelle

(A) $R_1$ représente H, $N(CH_3)_2$, $SO_3H$, $NO_2$ ou $SO_3^{\ominus}Na^{\oplus}$ et $R_2$, $R_3$, $R_4$, $R_5$ et $R_6$ représentent H; ou bien

(B) $R_1$, $R_4$, $R_5$ et $R_6$ représentent H et $R_2$ et $R_3$ représentent ensemble

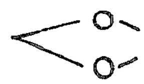

ou

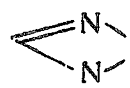

; ou bien

(C) $R_1$ et $R_4$ représentent $N(CH_3)_2$ et $R_2$, $R_3$, $R_5$ et $R_6$ représentent H; ou bien

(D) $R_1$, $R_2$, $R_3$ et $R_4$ représentent H et $R_5$ et $R_6$ représentent $OCH_3$,

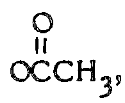

$OSi(CH_3)_2\ C_4H_9$ ou $N(CH_3)_2$; ou bien

(E) $R_1$, $R_4$, $R_5$ et $R_6$ représentent H et $R_2$ et $R_3$ représentent $OCH_3$, $SO_3H$ ou $CO_2H$ ou leurs sels; ou bien

(F) $R_1$, $R_3$, $R_4$, $R_5$ et $R_6$ représentent H et $R_2$ représente $(CH_3)_2N$.

**4.** Procédé suivant la revendication 3, caractérisé en ce que le naphtalène-2,3-dicarboxyaldéhyde substitué ou non substitué est de la formule :

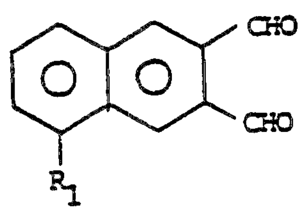

dans laquelle $R_1$ représente H, $N(CH_3)_2$, $SO_3H$, $NO_2$ ou $SO_3$ Na.

**5.** Procédé suivant la revendication 1, caractérisé en ce que le naphtalène-2,3-dicarboxyaldéhyde est de la formule :

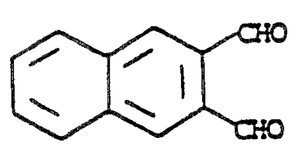

**6.** Procédé suivant l'une quelconque des revendications précédentes, caractérisé en ce que l'on fait réagir une solution 100 à 200 micromolaire du naphtalène-2,3-dicarboxyaldéhyde substitué ou non substitué avec une solution 100 à 200 micromolaire d'ion cyanure.

7. Procédé suivant l'une quelconque des revendications précédentes, caractérisé en ce que l'on réalise l'étape de réaction à un pH de 9 à 10.

8. Procédé suivant l'une quelconque des revendications précédentes, caractérisé en ce qu'une pluralité d'amines primaires sont présentes.

9. Procédé suivant la revendication 8, caractérisé en ce qu'il comprend en outre l'étape de séparation de la pluralité d'amines primaires en amines individuelles.

10. Procédé suivant la revendication 9, caractérisé en ce qu'on réalise l'étape de séparation par une chromatographie liquide à haute performance.

11. Procédé suivant l'une ou l'autre des revendications 8 et 9, caractérisé en ce qu'on réalise l'étape de séparation avant l'étape de réaction précitée.

12. Procédé suivant l'une ou l'autre des revendications 8 et 9, caractérisé en ce qu'on réalise l'étape de séparation après l'étape de réaction précitée.

13. Procédé suivant l'une quelconque des revendications 1 à 3 et 6 à 12, caractérisé en ce que le 2,3-naphtalène-dicarboxyaldéhyde est disubstitué ou tétrasubstitué.

14. Composé de la formule :

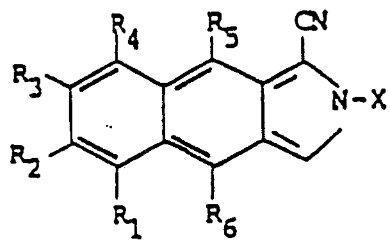

(A)

dans laquelle X est un radical provenant d'une amine primaire de la formule $X\text{-}NH_2$ et dans laquelle :

(A) $R_1$ représente H, $N(CH_3)_2$, $SO_3H$, $NO_2$ ou $SO^{\ominus}_3Na^{\oplus}$ et $R_2$, $R_3$, $R_4$, $R_5$ et $R_6$ représentent H; ou bien

(B) $R_1$, $R_4$, $R_5$ et $R_6$ représentent H et $R_2$ et $R_3$ représentent ensemble

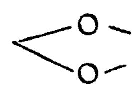

ou

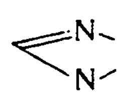

; ou bien

(C) $R_1$ et $R_4$ représentent $N(CH_3)_2$ et $R_2$, $R_3$, $R_5$ et $R_6$ représentent H; ou bien

(D) $R_1$, $R_2$, $R_3$ et $R_4$ représentent H et $R_5$ et $R_6$ représentent $OCH_3$,

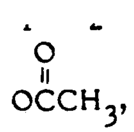

$OSi(CH_3)_2\ C_4H_9$ ou $N(CH_3)_2$; ou bien
(E) $R_1$, $R_4$, $R_5$ et $R_6$ représentent H et $R_2$ et $R_3$ représentent $OCH_3$, $SO_3H$ ou $CO_2H$ ou leurs sels; ou bien
(F) $R_1$, $R_3$, $R_4$, $R_5$ et $R_6$ représentent H et $R_2$ représente $(CH_3)_2N$.

**15.** Composé suivant la revendication 14, caractérisé en ce que $R_1$ représente H, $N(CH_3)_2$, $SO_3H$, $NO_2$ ou $SO_3^-Na^+$ et $R_2$, $R_3$, $R_4$, $R_5$ et $R_6$ représentent H.

**16.** Composé suivant la revendication 14, caractérisé en ce que $R_1$, $R_2$, $R_3$, $R_4$, $R_5$ et $R_6$ représentent chacun un atome d'hydrogène.

**17.** Composé suivant l'une quelconque des revendications 14, 15 et 16, caractérisé en ce que l'amine primaire de la formule $XNH_2$ est un peptide, un aminoacide ou une catécholamine.

**18.** Composé suivant l'une quelconque des revendications 14, 15 et 16, caractérisé en ce que l'amine primaire de la formule $XNH_2$ est de la glycine, de l'alanine, de la tyrosine, de la valine, de la phénylalanine, de l'acide aspartique, de la sérine, de l'acide glutamique, de l'histidine, de la thréonine, de l'isoleucine, de la méthionine, du tryptophane, de l'arginine, de l'asparagine, de la $\beta$-alanine, du GABA, de la N-acétyl-lysine, de la glycyl-glycine, de l'alanyl-alanine ou de l'aspartame.

Revendications pour l'Etat contractant suivant: AT

**1.** Procédé de dosage d'amines primaires comprenant les étapes suivantes:
(i) prévoir un naphtalène-2,3-dicarboxyaldéhyde substitué ou non substitué réactif avec des amines primaires;
(ii) faire réagir le naphtalène-2,3-dicarboxyaldéhyde substitué ou non substitué avec au moins un analyte d'amine primaire et un ion cyanure pour former un composé pouvant être soumis à des techniques de dosage fluorimétrique ou électrochimique;
(iii) doser fluorimétriquement ou électrochimiquement ce composé.

**2.** Procédé suivant la revendication 1, caractérisé en ce que ce composé est un fluorophore.

**3.** Procédé suivant l'une ou l'autre des revendications 1 et 2, caractérisé en ce que le naphtalène-2,3-dicarboxyaldéhyde substitué ou non substitué est de la formule :

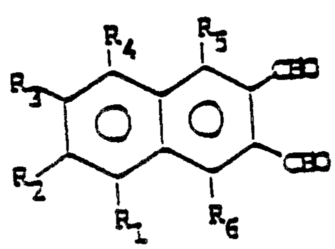

dans laquelle
(A) R· représente H, $N(CH_3)_2$, $SO_3H$, $NO_2$ ou $SO_3^{\ominus}Na^{\oplus}$ et $R_2$, $R_3$, $R_4$, $R_5$ et $R_6$ représentent H; ou bien
(B) R·, $R_4$, $R_5$ et $R_6$ représentent H et $R_2$ et $R_3$ représentent ensemble

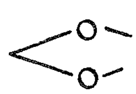

ou

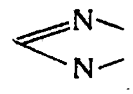

; ou bien
(C) $R_1$ et $R_4$ représentent $N(CH_3)_2$ et $R_2$, $R_3$, $R_5$ et $R_6$ représentent H; ou bien
(D) $R_1$, $R_2$, $R_3$ et $R_4$ représentent H et $R_5$ et $R_6$ représentent $OCH_3$,

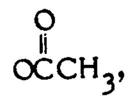

$OSi(CH_3)_2C_4H_9$ ou $N(CH_3)_2$; ou bien
(E) $R_1$, $R_4$, $R_5$ et $R_6$ représentent H et $R_2$ et $R_3$ représentent $OCH_3$, $SO_3H$ ou $CO_2H$ ou leurs sels; ou bien
(F) $R_1$, $R_3$, $R_4$, $R_5$ et $R_6$ représentent H et $R_2$ représente $(CH_3)_2N$.

4. Procédé suivant la revendication 3, caractérisé en ce que le naphtalène-2,3-dicarboxyaldéhyde substitué ou non substitué est de la formule :

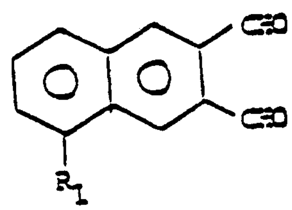

dans laquelle $R_1$ représente H, $N(CH_3)_2$, $SO_3H$, $NO_2$ ou $SO_3Na$.

5. Procédé suivant la revendication 1, caractérisé en ce que le naphtalène-2,3-dicarboxyaldéhyde est de la formule :

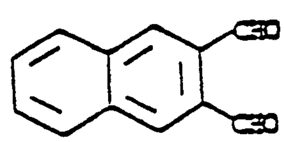

6. Procédé suivant l'une quelconque des revendications précédentes, caractérisé en ce que l'on fait réagir une solution 100 à 200 micromolaire du naphtalène-2,3-dicarboxyaldéhyde substitué ou non substitué avec une solution 100 à 200 micromolaire d'ion cyanure.

7. Procédé suivant l'une quelconque des revendications précédentes, caractérisé en ce que l'on réalise l'étape de réaction à un pH de 9 à 10.

8. Procédé suivant l'une quelconque des revendications précédentes, caractérisé en ce qu'une pluralité d'amines primaires sont présentes.

9. Procédé suivant la revendication 8, caractérisé en ce qu'il comprend en outre l'étape de séparation de la pluralité d'amines primaires en amines individuelles.

10. Procédé suivant la revendication 9, caractérisé en ce qu'on réalise l'étape de séparation par une

chromatographie liquide à haute performance.

**11.** Procédé suivant l'une ou l'autre des revendications 8 et 9, caractérisé en ce qu'on réalise l'étape de séparation avant l'étape de réaction précitée.

**12.** Procédé suivant l'une ou l'autre des revendications 8 et 9, caractérisé en ce qu'on réalise l'étape de séparation après l'étape de réaction précitée.

**13.** Procédé suivant l'une quelconque des revendications 1 à 3 et 6 à 12, caractérisé en ce que le 2,3-naphtalène-dicarboxyaldéhyde est disubstitué ou tétrasubstitué.

**14.** Procédé de fabrication d'un composé de formule générale :

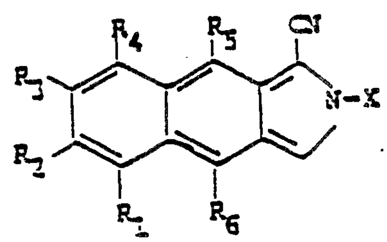

dans laquelle X est un radical provenant d'une amine primaire de la formule $X\text{-}NH_2$ et dans laquelle :

(A) $R_1$ représente H, $N(CH_3)_2$, $SO_3H$, $NO_2$ ou $SO^-_3$ $Na^+$ et $R_2$, $R_3$, $R_4$, $R_5$ et $R_6$ représentent H; ou bien

(B) $R_1$, $R_4$ $R_5$ et $R_6$ représentent H et $R_2$ et $R_3$ représentent ensemble

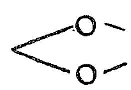

ou

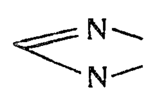

; ou bien

(C) $R_1$ et $R_4$ représentent $N(CH_3)_2$ et $R_2$, $R_3$, $R_5$ et $R_6$ représentent H; ou bien

(D) $R_1$, $R_2$, $R_3$ et $R_4$ représentent H et $R_5$ et $R_6$ représentent $OCH_3$,

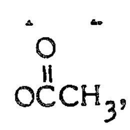

$OSi(CH_3)_2$ $C_4H_9$ ou $N(CH_3)_2$; ou bien

(E) $R_1$, $R_4$, $R_5$ et $R_6$ représentent H et $R_2$ et $R_3$ représentent $OCH_3$, $SO_3H$ ou $CO_2H$ ou leurs sels; ou bien

(F) $R_1$, $R_3$, $R_4$, $R_5$ et $R_6$ représentent H et $R_2$ représente $(CH_3)_2N$, lequel procédé comprend la réaction d'un dialdéhyde aromatique de formule générale

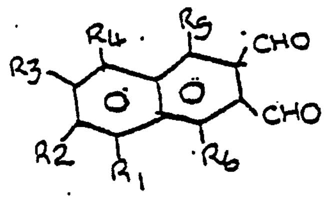

avec l'amine primaire en précense de cyanure.

15. Procédé suivant la revendication 14, caractérisé en ce que $R_1$ représente H, $N(CH_3)_2$, $SO_3H$, $NO_2$ ou $SO_3^-Na^+$ et $R_2$, $R_3$, $R_4$, $R_5$ et $R_6$ représentent H.

16. Procédé suivant la revendication 14, caractérisé en ce que $R_1$, $R_2$, $R_3$, $R_4$, $R_5$ et $R_6$ représentent chacun un atome d'hydrogène.

17. Procédé suivant l'une quelconque des revendications 14, 15 et 16, caractérisé en ce que l'amine primaire de la formule $XNH_2$ est un peptide, un aminoacide ou une catécholamine.

18. Procédé suivant l'une quelconque des revendications 14, 15 et 16, caractérisé en ce que l'amine primaire de la formule $XNH_2$ est de la glycine, de l'alanine, de la tyrosine, de la valine, de la phénylalanine, de l'acide aspartique, de la sérine, de l'acide glutamique, de l'histidine, de la thréonine, de l'isoleucine, de la méthionine, du tryptophane, de l'arginine, de l'asparagine, de la $\beta$-alanine, du GABA, de la N-acétyl-lysine, de la glycyl-glycine, de l'alanyl-alanine ou de l'aspartame.

## Ansprüche

1. Verfahren zur Untersuchung primärer Amine, welches die Schritte umfaßt:
   (i) Bereitstellung eines mit primären Aminen reagierenden substituierten oder unsubstituierten Naphtalin-2,3-dicarboxyaldehydes;
   (ii) Umsetzung des substituierten oder unsubstituierten Naphtalin-2,3-dicarboxyaldehydes mit wenigstens einem zu analysierenden primären Amin und Cyanidion zur Bildung einer fluorometrischen oder elektrochemischen Untersuchungstechniken zugänglichen Verbindung;
   (iii) fluorometrische oder elektrochemische Untersuchung der Verbindung.

2. Verfahren nach Anspruch 1, worin die Verbindung ein Fluorophor ist.

3. Verfahren nach Anspruch 1 oder 2, worin der substituierte oder unsubstituierte Naphtalin-2,3-dicarboxyaldehyd die Formel

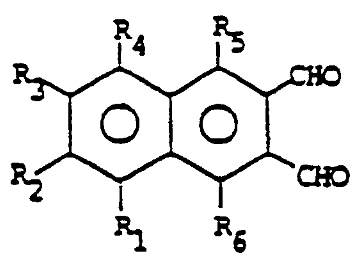

hat, worin
   (A) $R_1$ H, $N(CH_3)_2$, $SO_3H$, $NO_2$ oder $SO_3^{\ominus}NA^{\oplus}$ ist; und $R_2$, $R_3$, $R_4$, $R_5$ und $R_6$ H bedeuten; oder
   (B) $R_1$, $R_4$, $R_5$ und $R_6$ für H stehen, und $R_2$ und $R_3$ zusammen

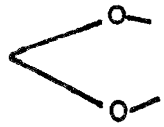

oder

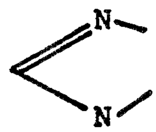

sind; oder
(C) $R_1$ und $R_4$ für $N(CH_3)_2$ stehen, und $R_2$, $R_3$, $R_5$ und $R_6$ H sind; oder
(D) $R_1$, $R_2$, $R_3$ und $R_4$ H bedeuten, und $R_5$ und $R_6$ $OCH_3$,

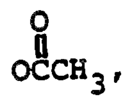

$OSi(CH_3)_2C_4H_9$ oder $N(CH_3)_2$ repräsentieren; oder
(E) $R_1$, $R_4$, $R_5$ und $R_6$ H sind, und $R_2$ und $R_3$ für $OCH_3$, $SO_3H$ oder $CO_2H$ oder die Salze davon stehen; oder
(F) $R_1$, $R_3$, $R_4$, $R_5$ und $R_6$ H bedeuten, und $R_2$ $(CH_3)_2N$ ist.

4. Verfahren nach Anspruch 3, worin der substituierte oder unsubstituierte Naphtalin-2,3-dicarboxyaldehyd die Formel

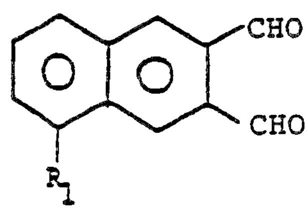

hat, worin $R_1$ H, $N(CH_3)_2$, $SO_3H$, $NO_2$ oder $SO_3$ Na ist.

5. Verfahren nach Anspruch 1, worin der substituierte oder unsubstituierte Naphtalin-2,3-dicarboxyaldehyd die Formel

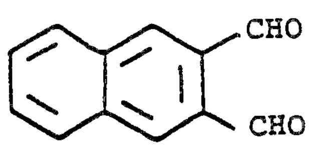

hat.

6. Verfahren nach irgendeinem der vorhergehenden Ansprüche, worin eine 100 bis 200 mikromolare

Lösung des substituierten oder unsubstituierten Naphtalin-2,3-dicarboxyaldehydes mit einer 100 bis 200 mikromolaren Lösung von Cyanidion umgesetzt wird.

7. Verfahren nach irgendeinem der vorhergehenden Ansprüche, worin der Reaktionsschritt bei einem pH-Wert von 9 bis 10 durchgeführt wird.

8. Verfahren nach irgendeinem der vorhergehenden Ansprüche, worin eine Vielzahl von primären Aminen anwesend sind.

9. Verfahren nach Anspruch 8, welches weiters den Schritt der Auftrennung der Vielzahl von primären Aminen in einzelne Amine umfaßt.

10. Verfahren nach Anspruch 9, worin der Schritt der Auftrennung mittels Hochleistungsflüssigkeitschromatographie durchgeführt wird.

11. Verfahren nach den Ansprüchen 8 oder 9, worin der Schritt der Auftrennung vor dem Reaktionsschritt durchgeführt wird.

12. Verfahren nach den Ansprüchen 8 oder 9, worin der Schritt der Auftrennung nach dem Reaktionsschritt durchgeführt wird.

13. Verfahren nach irgendeinem der Ansprüche 1 bis 3 und 6 bis 12, worin der 2,3-Naphtalin-dicarboxyaldehyd disubstituiert oder tetrasubstituiert ist.

14. Verbindung der Formel:

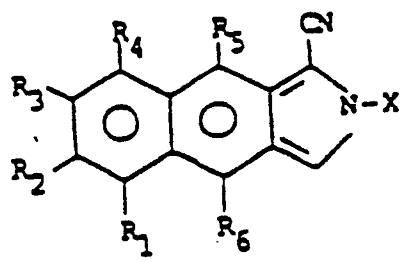

(A),

worin X ein von einem primären Amin der Formel $X\text{-}NH_2$ abgeleiteter Rest ist, und worin

(A) $R_1$ H, $N(CH_3)_2$, $SO_3H$, $NO_2$ oder $SO_3^{\ominus}Na^{\oplus}$ bedeutet, und $R_2$, $R_3$, $R_4$, $R_5$ und $R_6$ H sind; oder

(B) $R_1$, $R_4$, $R_5$ und $R_6$ für H stehen, und $R_2$ und $R_3$ zusammen

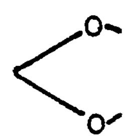

oder

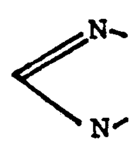

sind; oder

(C) $R_1$ und $R_4$ $N(CH_3)_2$ repräsentieren, und $R_2$, $R_3$, $R_5$ und $R_6$ für H stehen; oder

(D) $R_4$, $R_2$, $R_3$ und $R_4$ H sind, und $R_5$ und $R_6$ $OCH_3$,

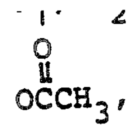

$OSi(CH_3)_2C_4H_9$ oder $N(CH_3)_2$ bedeuten; oder

(E) $R_1$, $R_4$, $R_5$ und $R_6$ für H stehen, und $R_2$ und $R_3$ $OCH_3$, $SO_3H$ oder $CO_2H$ oder die Salze davon repräsentieren; oder

(F) $R_1$, $R_3$, $R_4$, $R_5$ und $R_6$ H bedeuten, und $R_2$ $(CH_3)_2N$ ist.

**15.** Verbindung nach Anspruch 14, worin $R_1$ für H, $N(CH_3)_2$, $SO_3H$, $NO_2$ oder $SO_3^-Na^+$ steht, und $R_2$, $R_3$, $R_4$, $R_5$ und $R_6$ H bedeuten.

**16.** Verbindung nach Anspruch 14, worin jedes von $R_1$, $R_2$, $R_3$, $R_4$, $R_5$ und $R_6$ ein Wasserstoffatom ist.

**17.** Verbindung nach Anspruch 14, 15 oder 16, worin das primäre Amin der Formel $XNH_2$ ein Peptid, eine Aminosäure oder ein Catecholamin ist.

**18.** Verbindung nach den Ansprüchen 14, 15 oder 16, worin das primäre Amin der Formel $XNH_2$ Glycin, Alanin, Tyrosin, Valin, Phenylalanin, Asparaginsäure, Serin, Glutaminsäure, Histidin, Threonin, Isoleucin, Methionin, Tryptophan, Arginin, Asparagin, ß-Alanin, GABA, N-Acetyl-lysin, Glycyl-glycin, Alanyl-alanin oder Aspartam ist.

Patentansprüche für folgenden Vertragsstaat: AT

**1.** Verfahren zur Untersuchung primärer Amine, welches die Schritte umfaßt:

(1) Bereitstellung eines mit primären Aminen reagierenden substituierten oder unsubstituierten Naphtalin-2,3-dicarboxyaldehydes;

(ii) Umsetzung des substituierten oder unsubstituierten Naphtalin-2,3-dicarboxyaldehydes mit wenigstens einem zu analysierenden primären Amin und Cyanidion zur Bildung einer fluorometrischen oder elektrochemischen Untersuchungstechniken zugänglichen Verbindung;

(iii) fluorometrische oder elektrochemische Untersuchung der Verbindung.

**2.** Verfahren nach Anspruch 1, worin die Verbindung ein Fluorophor ist.

**3.** Verfahren nach Anspruch 1 oder 2, worin der substituierte oder unsubstituierte Naphtalin-2,3-dicarboxyaldehyd die Formel

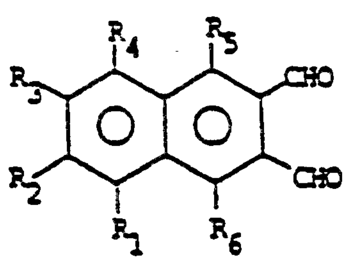

hat, worin

(A) $R_1$ H, $N(CH_3)_2$, $SO_3H$, $NO_2$ oder $SO_3^{\ominus}NA^{\oplus}$ ist; und $R_2$, $R_3$, $R_4$, $R_5$ und $R_6$ H bedeuten; oder

(B) $R_1$, $R_4$, $R_5$ und $R_6$ für H stehen, und $R_2$ und $R_3$ zusammen

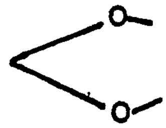

oder

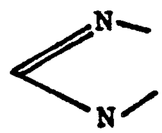

sind; oder
(C) $R_1$ und $R_4$ für $N(CH_3)_2$ stehen, und $R_2$, $R_3$, $R_5$ und $R_6$ H sind; oder
(D) $R_1$, $R_2$, $R_3$ und $R_4$ H bedeuten, und $R_5$ und $R_6$ $OCH_3$,

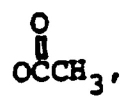

$OSi(CH_3)_2C_4H_9$ oder $N(CH_3)_2$ repräsentieren; oder
(E) $R_1$, $R_4$, $R_5$ und $R_6$ H sind, und $R_2$ und $R_3$ für $OCH_3$, $SO_3H$ oder $CO_2H$ oder die Salze davon stehen; oder
(F) $R_1$, $R_3$, $R_4$, $R_5$ und $R_6$ H bedeuten, und $R_2$ $(CH_3)_2N$ ist.

4. Verfahren nach Anspruch 3, worin der substituierte oder unsubstituierte Naphtalin-2,3-dicarboxyaldehyd die Formel

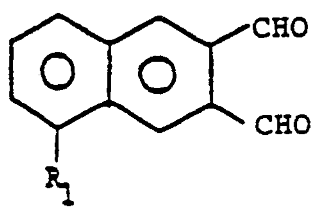

hat, worin $R_1$ H, $N(CH_3)_2$, $SO_3H$, $NO_2$ oder $SO_3$ Na ist.

5. Verfahren nach Anspruch 1, worin der substituierte oder unsubstituierte Naphtalin-2,3-dicarboxyaldehyd die Formel

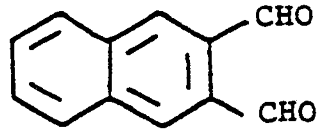

hat.

6. Verfahren nach irgendeinem der vorhergehenden Ansprüche, worin eine 100 bis 200 mikromolare Lösung des substituierten oder unsubstituierten Naphtalin-2,3-dicarboxyaldehydes mit einer 100 bis

200 mikromolaren Lösung von Cyanidion umgesetzt wird.

7. Verfahren nach irgendeinem der vorhergehenden Ansprüche, worin der Reaktionsschritt bei einem pH-Wert von 9 bis 10 durchgeführt wird.

8. Verfahren nach irgendeinem der vorhergehenden Ansprüche, worin eine Vielzahl von primären Aminen anwesend sind.

9. Verfahren nach Anspruch 8, welches weiters den Schritt der Auftrennung der Vielzahl von primären Aminen in einzelne Amine umfaßt.

10. verfahren nach Anspruch 9, worin der Schritt der Auftrennung mittels Hochleistungsflüssigkeitschromatographie durchgeführt wird.

11. Verfahren nach den Ansprüchen 8 oder 9, worin der Schritt der Auftrennung vor dem Reaktionsschritt durchgeführt wird.

12. Verfahren nach den Ansprüchen 8 oder 9, worin der Schritt der Auftrennung nach dem Reaktionsschritt durchgeführt wird.

13. Verfahren nach irgendeinem der Ansprüche 1 bis 3 und 6 bis 12, worin der 2,3-Naphtalin-dicarboxyaldehyd disubstituiert oder tetrasubstituiert ist.

14. Verfahren zur Herstellung einer Verbindung der allgemeinen Formel:

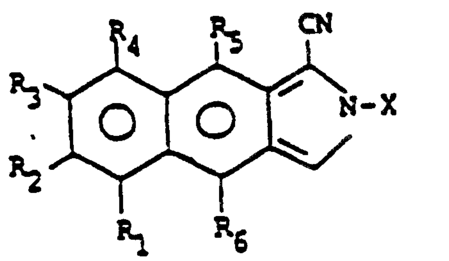

(A),

worin X ein von einem primären Amin der Formel $X\text{-}NH_2$ abgeleiteter Rest ist, und worin
(A) $R_1$ H, $N(CH_3)_2$, $SO_3H$, $NO_2$ oder $SO_3^{\ominus}Na^{\oplus}$ bedeutet, und $R_2$, $R_3$, $R_4$, $R_5$ und $R_5$ H sind; oder
(B) $R_1$, $R_4$, $R_5$ und $R_6$ für H stehen, und $R_2$ und $R_3$ zusammen

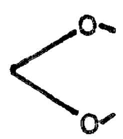

oder

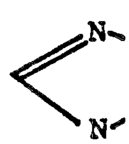

sind; oder
(C) $R_1$ und $R_4$ $N(CH_3)_2$ repräsentieren, und $R_2$, $R_3$, $R_5$ und $R_6$ für H stehen; oder
(D) $R_1$, $R_2$, $R_3$ und $R_4$ H sind, und $R_5$ und $R_6$ $OCH_3$,

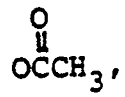

$OSi(CH_3)_2C_4H_9$ oder $N(CH_3)_2$ bedeuten; oder
(E) $R_1$, $R_4$, $R_5$ und $R_6$ für H stehen, und $R_2$ und $R_3$ $OCH_3$, $SO_3H$ oder $CO_2H$ oder die Salze davon repräsentieren; oder
(F) $R_1$, $R_3$, $R_4$, $R_5$ und $R_6$ H bedeuten, und $R_2$ $(CH_3)_2N$ ist welches Verfahren die Umsetzung eines aromatischen Dialdehyds der allgemeinen Formel

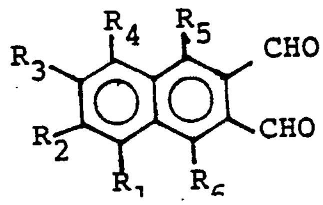

mit dem primären Amin in Anwesenheit von Cyanid umfaßt.

**15.** Verfahren nach Anspruch 14, worin $R_1$ für H, $N(CH_3)_2$, $SO_3H$, $NO_2$ oder $SO_3^-Na^+$ steht, und $R_2$, $R_3$, $R_4$, $R_5$ und $R_6$ H bedeuten.

**16.** Verfahren nach Anspruch 14, worin jedes von $R_1$, $R_2$, $R_3$, $R_4$, $R_5$ und $R_6$ ein Wasserstoffatom ist.

**17.** Verfahren nach Anspruch 14, 15 oder 16, worin das primäre Amin der Formel $XNH_2$ ein Peptid, eine Aminosäure oder ein Catecholamin ist.

**18.** Verfahren nach den Ansprüchen 14, 15 oder 16, worin das primäre Amin der Formel $XNH_2$ Glycin, Alanin, Tyrosin, Valin, Phenylalanin, Asparaginsäure, Serin, Glutaminsäure, Histidin, Threonin, Isoleucin, Methionin, Tryptophan, Arginin, Asparagin, $\beta$-Alanin, GABA, N-Acetyl-lysin, Glycyl-glycin, Alanyl-alanin oder Aspartam ist.

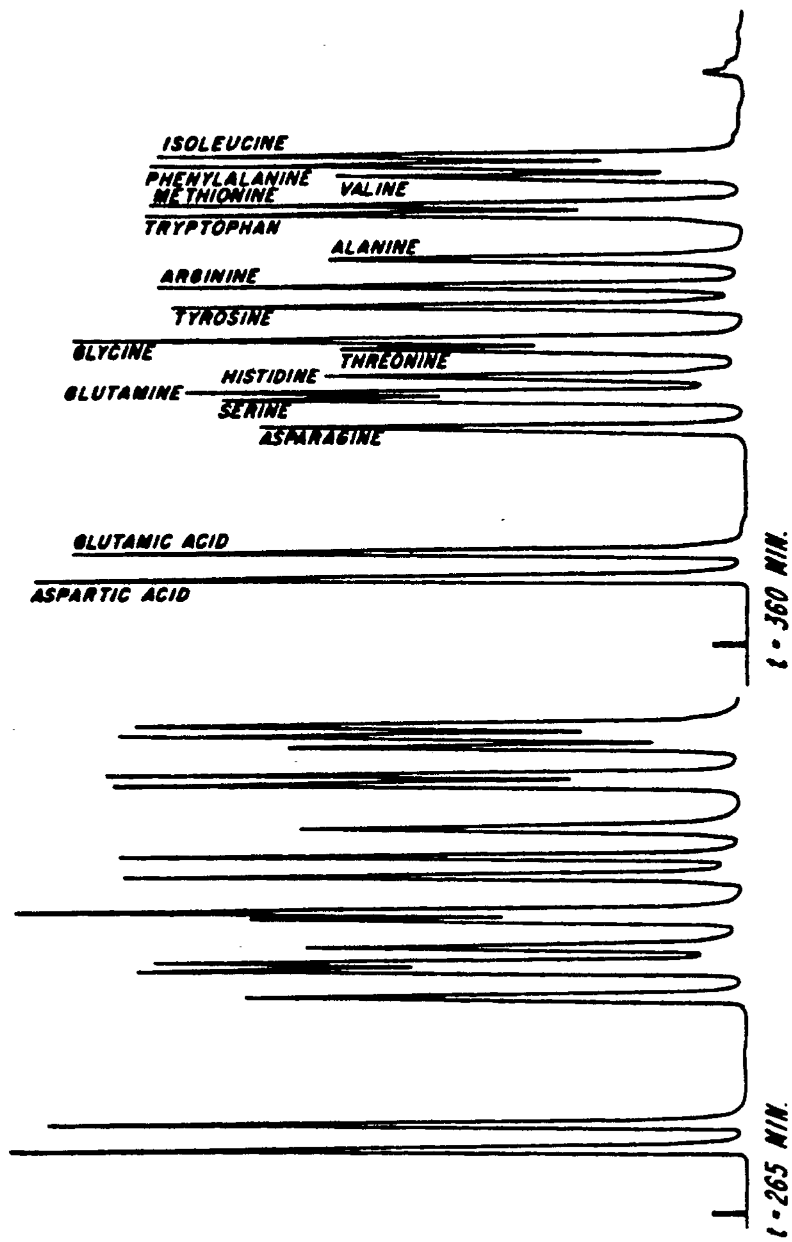
ISOLEUCINE
PHENYLALANINE
METHIONINE
VALINE
TRYPTOPHAN
ALANINE
ARGININE
TYROSINE
GLYCINE
THREONINE
HISTIDINE
GLUTAMINE
SERINE
ASPARAGINE
GLUTAMIC ACID
ASPARTIC ACID
t - 360 MIN.
t - 265 MIN.

FIG-1

Fig. 2

NDA-CN-ASPARTAME IN BORATE BUFFER
pH = 9.5

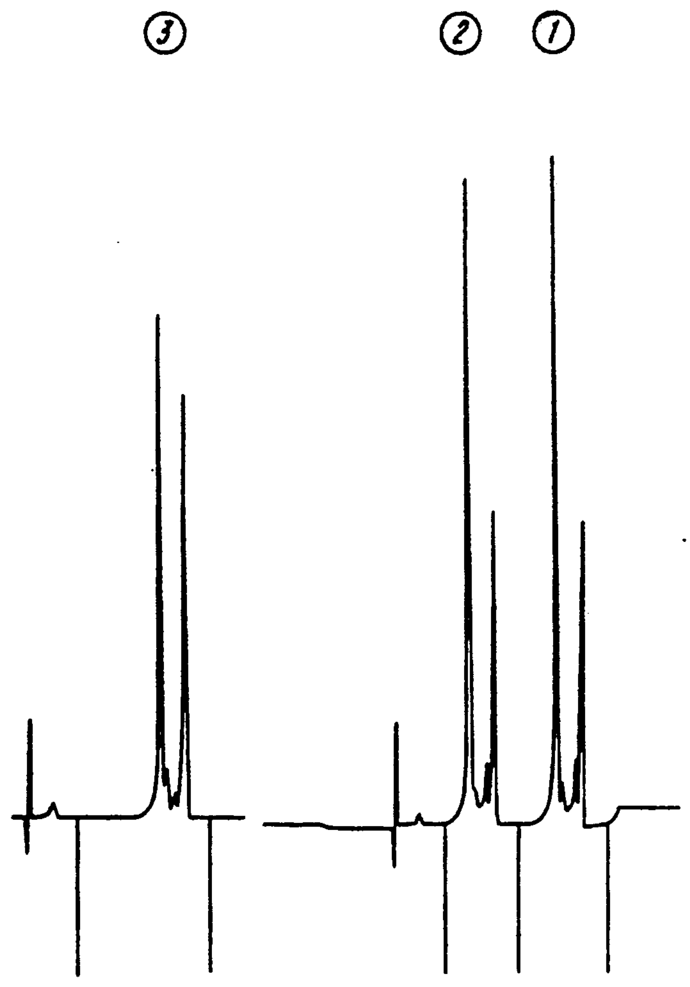

Fig. 3

DEPENDENCE OF THE PSEUDO-FIRST ORDER RATE CONSTANT FOR THE FORMATION OF 1-CYANO 2-ALANYL BENZO-ISOINDOLE UPON THE EFFECTIVE CONCENTRATION OF CYANIDE AT pH 9.5 IN BORATE BUFFER AT 25°C AND IONIC STRENGTH 0.1

[NDA] : $2.0 \times 10^{-4}$ M.
[ALANINE] : $2.0 \times 10^{-6}$ M.

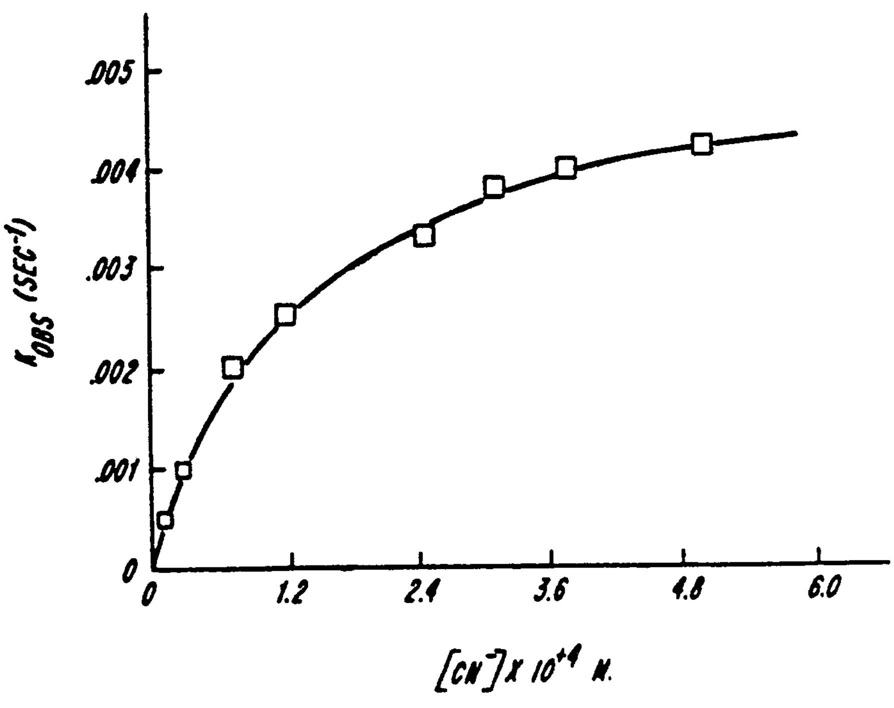

FIG. 4

DEPENDENCE OF THE PSEUDO-FIRST ORDER RATE CONSTANT FOR THE FORMATION OF 1-CYANO 2-ALANYL BENZO-ISOINDOLE UPON THE CONCENTRATION OF NAPHTHALENE DIALDEHYDE AT pH 9.5 IN BORATE BUFFER AT 25°C AND IONIC STRENGTH 0.1

[CYANIDE] TOTAL : $1.0 \times 10^{-3}$ M
[ALANINE] TOTAL : $2.0 \times 10^{-6}$ M

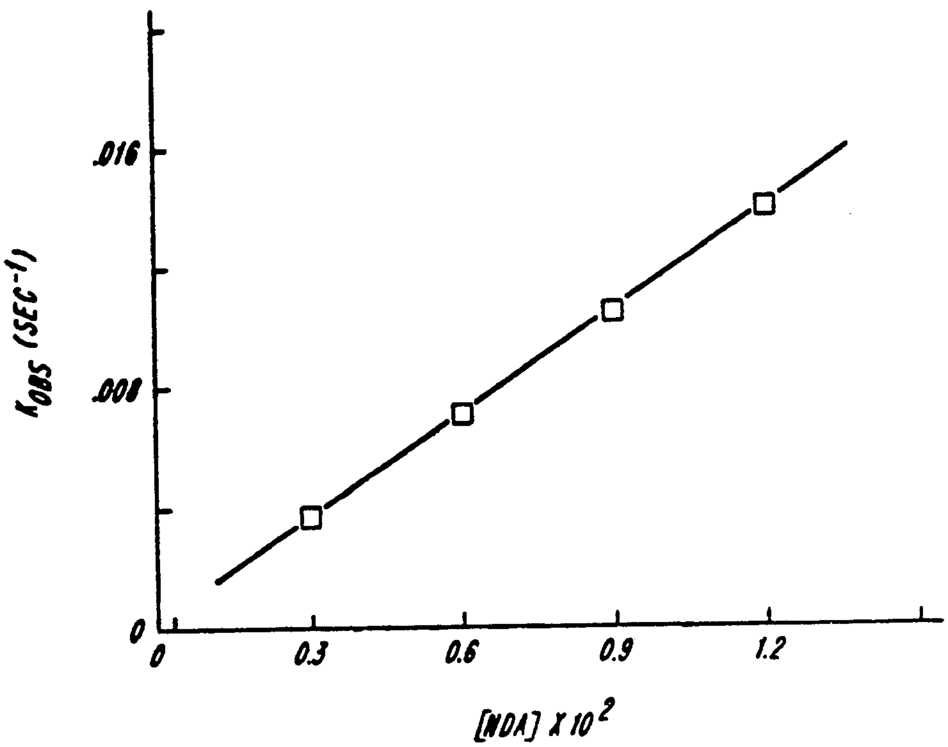

FIG. 5

EXCITATION SPECTRUM

NDA/CN/ALANINE

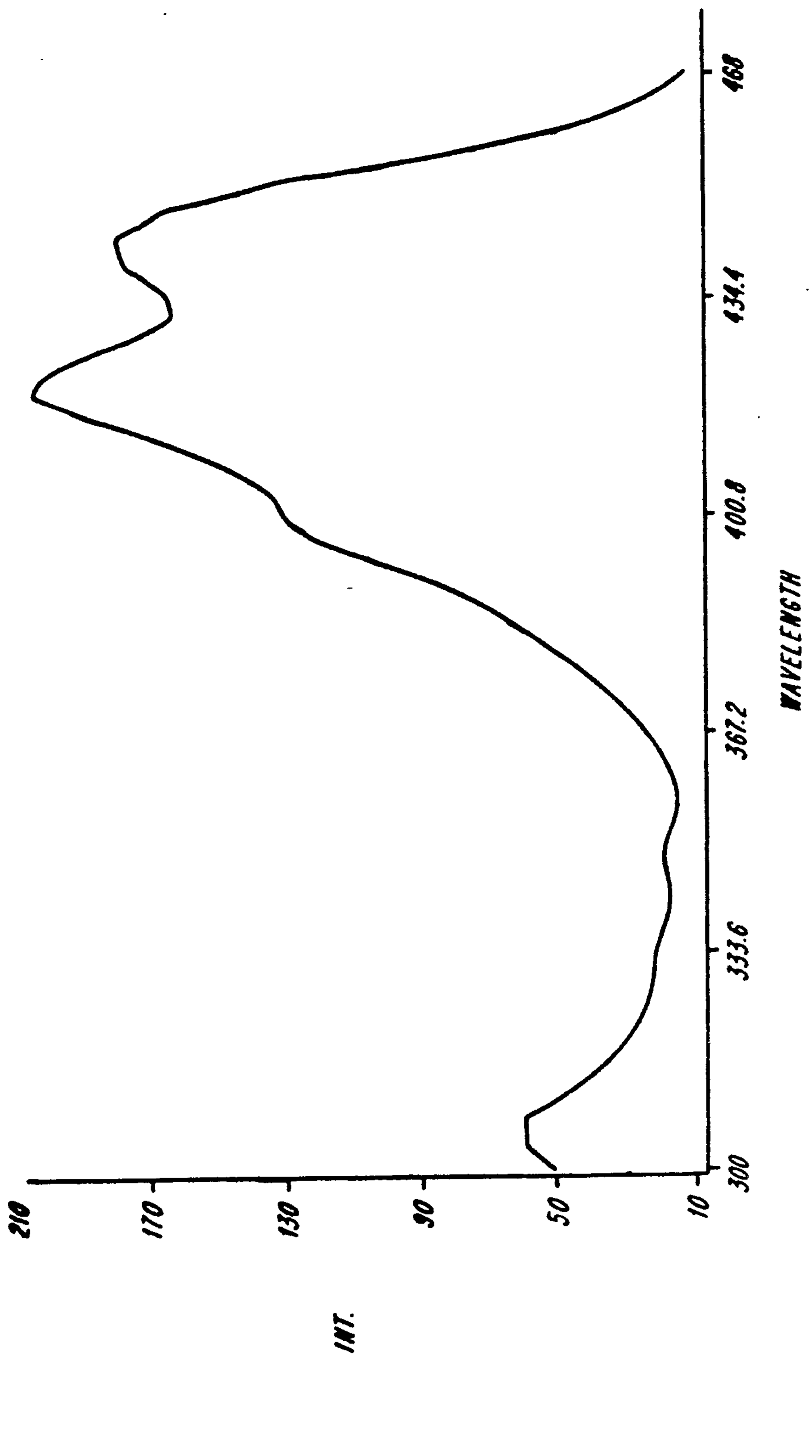

Fig. 6

EMISSION SPECTRUM

NDA/CN/ALANINE

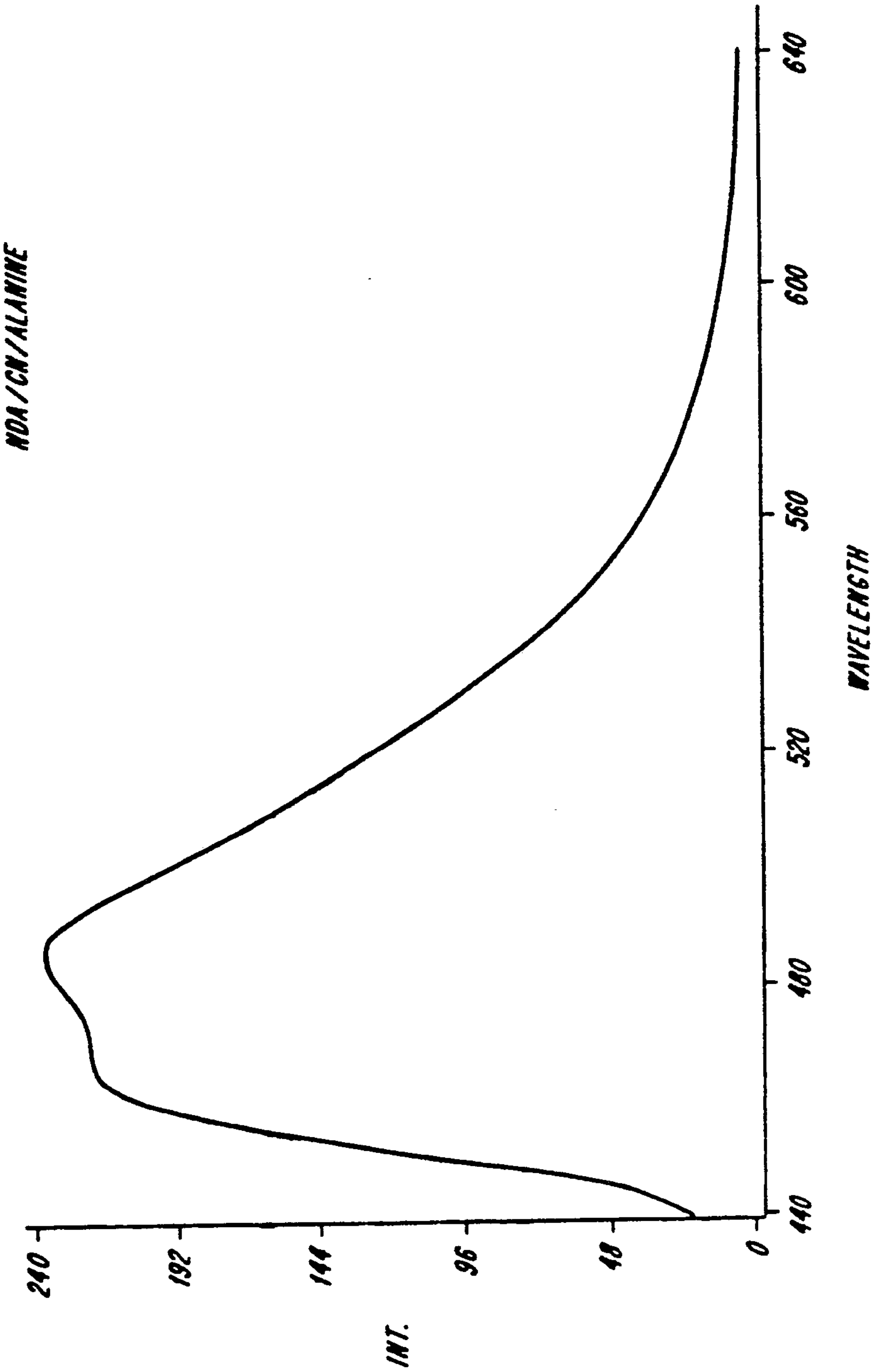

Fig. 7

pH-RATE PROFILE FOR THE FORMATION OF 1-CYANO 2-ALANYL BENZO-ISOINDOLE MAINTAINED BY A pH-STAT AT 30°C AND IONIC STRENGTH 0.1

[NDA] TOTAL : 2.0 X $10^{-4}$ M.
[CYANIDE] TOTAL : 2.0 X $10^{-4}$ M.
[ALANINE] TOTAL : 1.0 X $10^{-6}$ M.

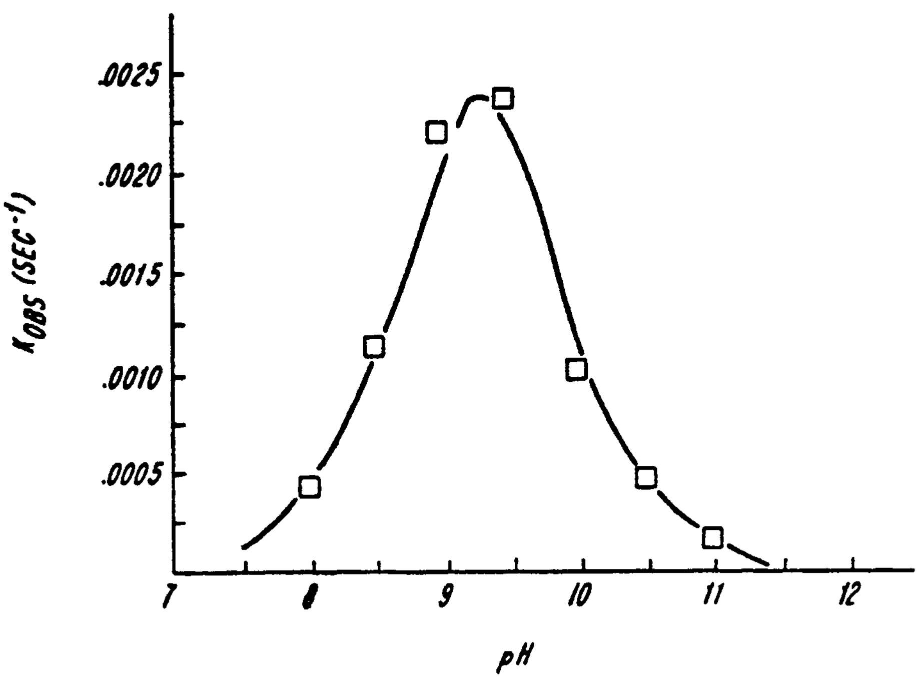

Fig. 8

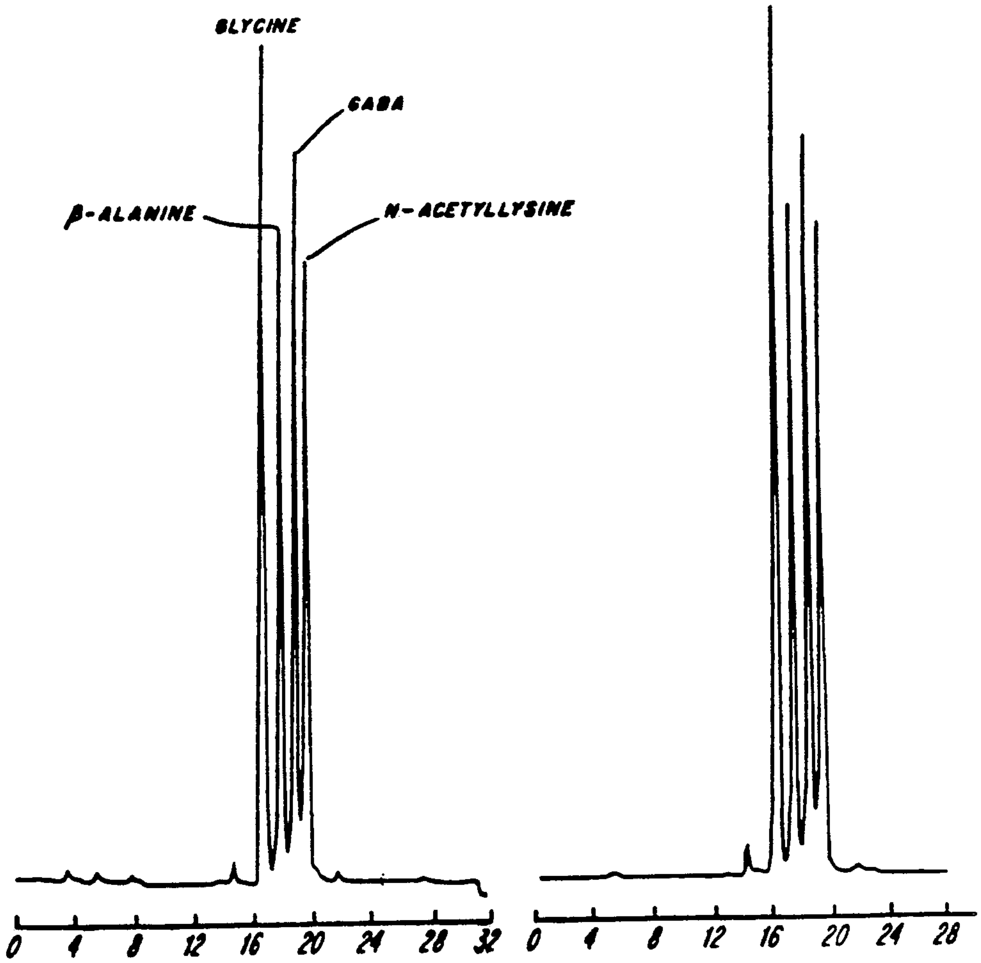


t = 15 MINUTES

t = 3 HOURS

Fig. 9

BENZOISOINDOLE PRODUCTS OF AMINO ACID MIXTURES

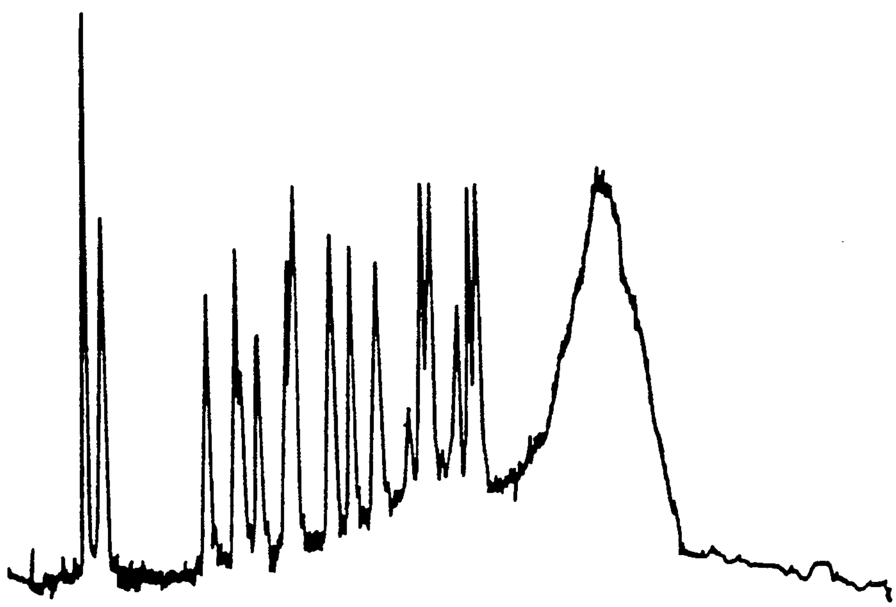

Fig. 10

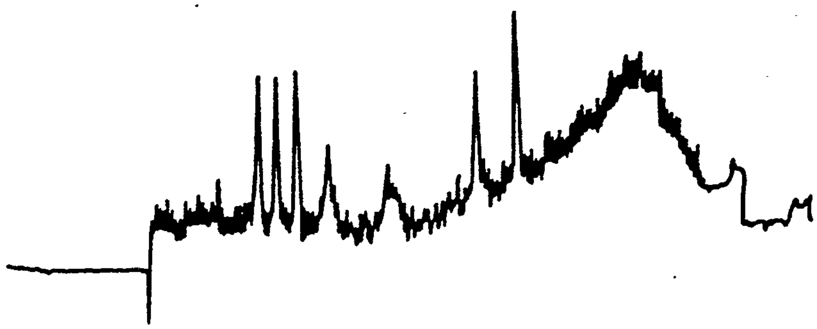

500 FEMTOMOLES MG. WITH OPA/ME R = 0.2 $\mu$A

[OPA] : 1.8 X $10^{-6}$ M.
[ME] : 10 X $10^{-6}$ M. IN 0.1 M SODIUM BORATE

$\lambda$EXC. = 230nm
$\lambda$EM. >418nm

Fig. 11

DEGRADATION PROFILE OF:

A – 1-CYANO 2-GLYCYL BENZO-ISOINDOLE
B – 1-MERCAPTOETHANOL 2-GLYCYL ISOINDOLE

IN A MIXED SOLVENT, PROTECTED FROM THE LIGHT, CONSISTING OF 20% METHANOL AND 80% BORATE BUFFER ($\mu$ = 0.1 M., pH = 9.5) AT 25°C, MONITORED BY HPLC

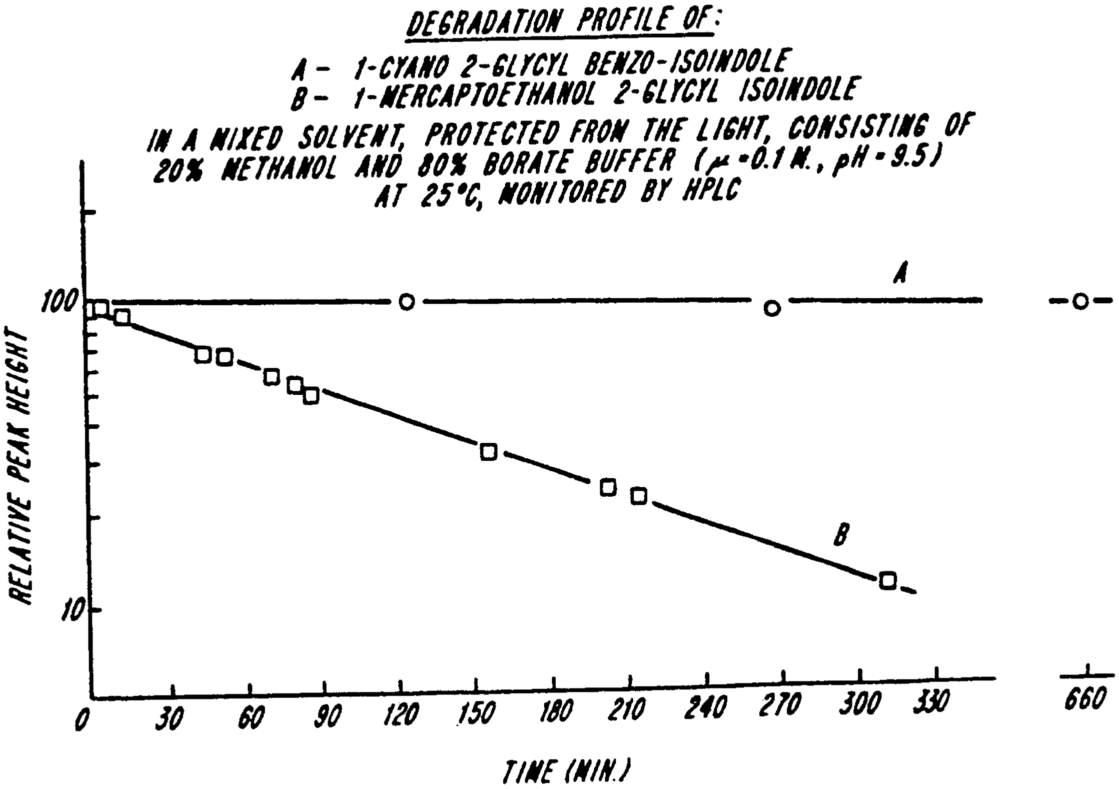

Fig. 12

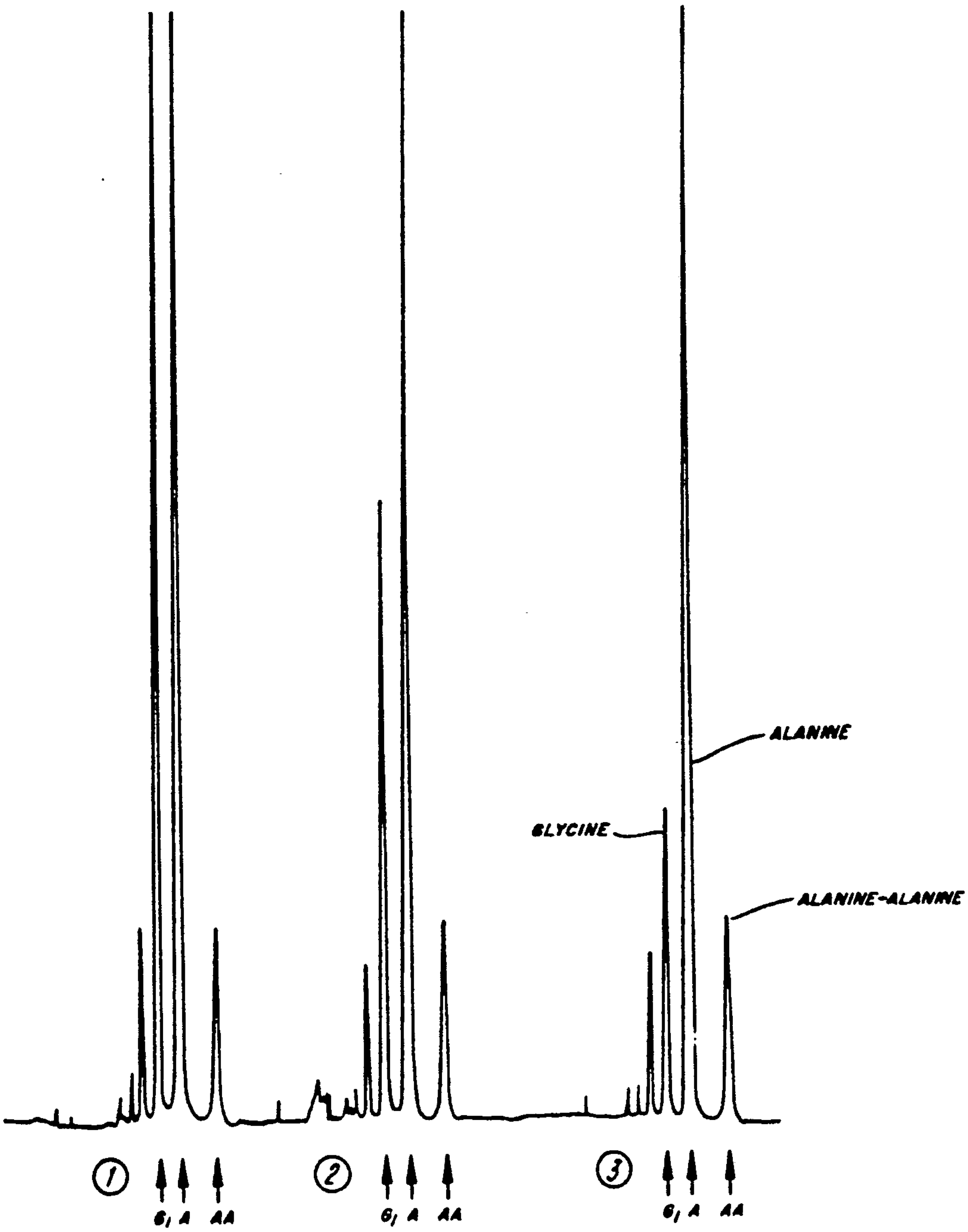


20 PICOMOLES INJECTION OF
GLY+ALA+ALA-ALA MIXTURE WITH OPA/ME AT:

pH = 9.5
λEXC = 335nm
λEM = 430nm
R = 0.01

FIG. 13

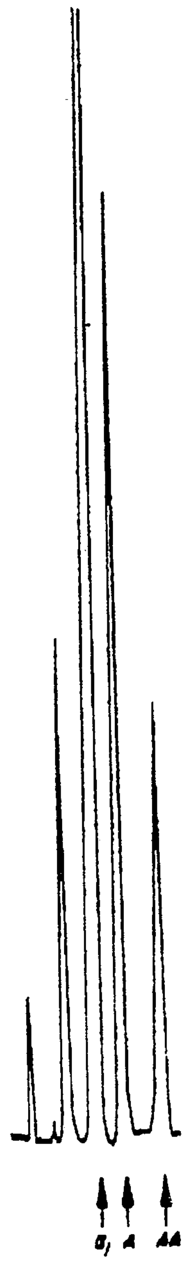


4 PICOMOLES INJECTION 20λ OF 0.2 X $10^{-6}$ M. SOLUTION OF GLY + ALA + ALA-ALA $\bar{C}$ NDA/CN AT:

pH = 9.5
λEXC = 420nm
λEM = 480nm
R = 0.01